(19) Europäisches Patentamt
European Patent Office
Office européen des brevets

(11) Publication number: 0 087 792 A1

# (12) EUROPEAN PATENT APPLICATION

(21) Application number: 83101923.7

(22) Date of filing: 28.02.83

(51) Int. Cl.³: C 07 D 499/00
A 61 K 31/43

(30) Priority: 01.03.82 US 353453
29.04.82 US 373088

(43) Date of publication of application:
07.09.83 Bulletin 83/36

(84) Designated Contracting States:
CH DE FR GB IT LI NL

(71) Applicant: MERCK & CO. INC.
126, East Lincoln Avenue P.O. Box 2000
Rahway New Jersey 07065(US)

(72) Inventor: Leanza, William J.
20 Rutherford Road
Berkeley Heights New Jersey 07922(US)

(72) Inventor: Christensen, Burton G.
770 Anderson Avenue Apt. 19H
Cliffside Park New Jersey 07010(US)

(72) Inventor: Dininno, Frank P.
46 Bentley Avenue
Old Bridge New Jersey 08857(US)

(72) Inventor: Ratcliffe, Ronald W.
234 Matawan Avenue
Matawan New Jersey 07747(US)

(74) Representative: Abitz, Walter, Dr.-Ing. et al,
Abitz, Morf, Gritschneder, Freiherr von Wittgenstein
Postfach 86 01 09
D-8000 München 86(DE)

## (54) 6-Substituted-2-carbamimidoyl-pen-2-em-3-carboxylic acids, process for preparing them and antibiotic compositions comprising them.

(57) Disclosed are 6-substituted-2-carbamimidoyl-pen-2-em-3-carboxylic acids (I) having the representative structure:

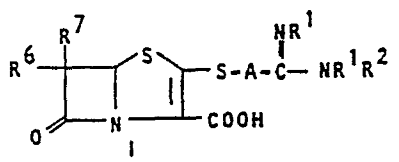

I

wherein $R^6$, and $R^7$ are, *inter alia*, independently selected from the group consisting of hydrogen, alkyl, alkoxyl, halo, OH, COOH, alkenyl, aryl and aralkyl; A is a direct, single bond connecting the indicated S and C atoms, or A is a cyclic or acyclic connecting group selected, *inter alia*, from alkyl, cycloalkyl, aryl, heteroaryl, heteroalkyl; $R^1$ and $R^2$, which define the carbamimidoyl function, are, *inter alia*, independently selected from hydrogen, alkyl, aryl; additionally, said carbamimidoyl is characterized by cyclic structures achieved by the joinder of the two nitrogen atoms *via* their substituents and by their joinder to connecting group A; additionally, "carbamimidiums" are disclosed by quarternization of one of the nitrogen atoms of said carbamimidoyl.

Such compounds are useful as antibiotics. Also disclosed are processes for the preparation of such compounds.

EP 0 087 792 A1

0087792

16747 Y

## TITLE OF THE INVENTION

6-SUBSTITUTED-2-CARBAMIMIDOYL-PEN-2-EM-3-CARBOXYLIC ACIDS, PROCESS FOR PREPARING THEM AND ANTIBIOTIC COMPOSITIONS COMPRISING THEM.

## BACKGROUND OF THE INVENTION

This invention relates to 6-substituted-2-carbamimidoyl-pen-2-em-3-carboxylic acids (I) and the pharmaceutically acceptable salt, ester and amide derivatives thereof which are useful as antibiotics:

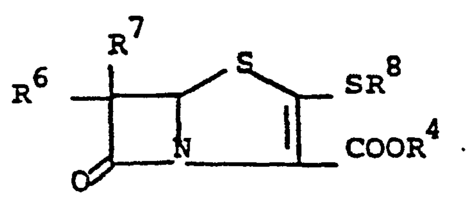

I

wherein $R^6$ and $R^7$ are independently selected from the group consisting of: hydrogen; substituted and unsubstituted: alkyl, alkoxyl, alkenyl, and alkynyl, having from 1-10 carbon atoms; halo (especially chloro and fluoro); hydroxyl; carboxyl; cycloalkyl,

cycloalkylalkyl and alkylcycloalkyl, having 3-6 carbon atoms in the cycloalkyl ring and 1-6 carbon atoms in the alkyl moieties; aryl such as phenyl; aralkyl, aralkenyl, and aralkynyl wherein the aryl moiety is phenyl and the aliphatic portion has 1-6 carbon atoms; heteroaryl, heteroaralkyl, heteroalkyl, heterocyclyl and heterocyclylalkyl; wherein the heteroatom or atoms are selected from O, N and S; wherein the substituent or substituents on $R^6$ and $R^7$ are independently selected from chloro, fluoro, carboxyl, amino, R°NH, $R°_2N$ (R° is $C_{1-6}$ alkyl); bromo, hydroxy, and alkoxyl having 1-6 carbon atoms; additionally $R^6$ and $R^7$ may be joined to form, together with the carbon atom to which they are attached, a cyclicalkyl having 3-6 carbon atoms.

$R^8$ is generically defined to be a "carbamimidoyl", which may be defined by the following structures:

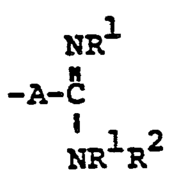

and

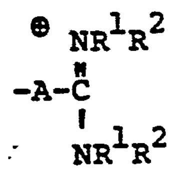

wherein A, the cyclic or acylic connecting group, and $R^1$ and $R^2$ are defined below. The definition of $R^8$ also embraces cyclic structures, which may be generically represented, for example, thusly:

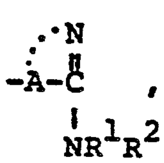 ,

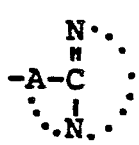 ,

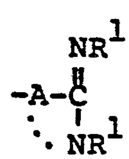 ,

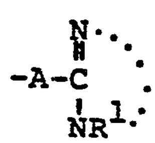 , and

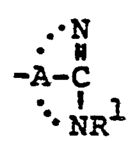

wherein the dotted lines indicate that the nitrogen atoms of the so-called carbamimidoyl function may participate in the formation of the cyclic structures indicated above. Representative specific embodiments for $R^8$ (as well as $R^6$ $R^7$) follow, but, in the generic sense, the components: $R^1$, $R^2$ and A which comprise $R^8$ are defined, thusly:

A, the cyclic or acyclic connector, is selected from the group consisting of alkyl, alkenyl, and alkynyl having 1-10 carbon atoms which may be interrupted by a hetero atom selected from O, S or N, or by a ring such as phenyl, cycloalkyl, cycloalkenyl, heterocyclyl or heteroaryl wherein such cyclic interruptions comprise 3-6 ring atoms selected from C, O, S and N; cycloalkyl, cycloalkenyl having 3-6 carbon atoms; heterocyclyl; heteroaryl; and phenyl; A also represents a direct, single bond connecting the indicated S and C atoms.

$R^1$ and $R^2$ are independently selected from hydrogen, alkoxyl, amino, mono- and disubstituted amino, and the previously defined values for the group A, such as: alkyl, aryl, cycloalkyl, heteroarlkyl, alkylaryl, alkylarylalkyl, and heterocyclyl and heteroaryl.

$R^4$ is hydrogen, a removable protecting group, a synthetically useful salt moiety, or a pharmaceutically acceptable salt or ester moiety.

This invention particularly relates to 6-(1-hydroxyethyl-2-carbamimidoyl-pen-2-em-3-carboxylic acids (Ia) and the pharmaceutically acceptable salt, ester and amide derivatives thereof which are useful as antibiotics:

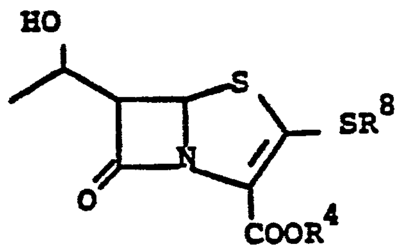

Ia

wherein: $R^4$ and $R^8$ are as defined above.

It should be noted that the final products of this invention (I) can exist in either neutral or zwitterionic (internal salt) forms. In the zwitterionic form, the basic function is protonated and positively charged and the carboxyl group is deprotonated and negatively charged. The zwitterionic form is the predominant species under most conditions and is in equilibrium with a minor

amount of the uncharged, neutral species. The equilibrium process is conveniently visualized as an internal acid-base neutralization. The neutral and zwitterionic forms are shown below.

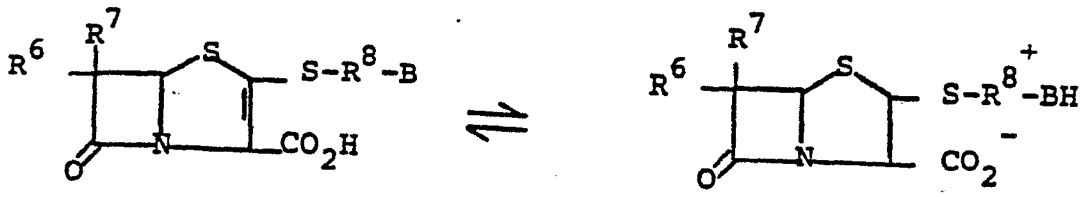

wherein B is the carbamimidoyl group.

Further, the final products of this invention I wherein $R^8$ contains a positively charged quaternary nitrogen function such as the "carbamimidinium" can exist as zwitterionic (internal salt) forms or as external salt forms. The preferred form of this product group is the zwitterionic or internal salt form. These forms are shown below:

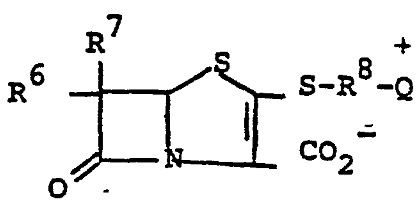

Zwitterionic (internal salt) Form

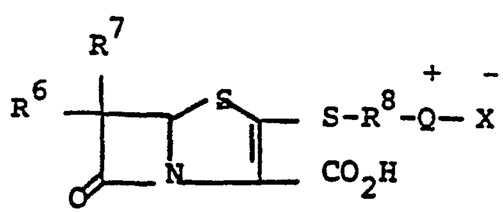

External Salt Form

wherein Q represents the quaterized nitrogen group, and wherein X is a pharmaceutically acceptable anion such as those listed in U.S. Patent 4,194,047, issued 3/18/80, which is incorporated herein by reference.

This invention also relates to the carboxyl derivatives of I which are antibiotics and which may be represented by the following generic structure (Ib):

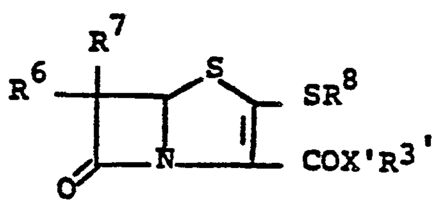

Ib

wherein X' is oxygen, sulphur or NR' (R' = H or lower alkyl having 1-6 carbon atoms); and $R^{3'}$ is, hydrogen, or, <u>inter alia</u> is representatively selected to provide the pharmaceutically acceptable salt, ester, anhydride ($R^{3'}$ is acyl), and amide moieties known in bicyclic ß-lactam antibiotic art; $R^{3'}$ may also be a readily removable blocking group. The definition of $R^{3'}$ is given in greater detail below.

This invention also relates to processes for the preparation of such compounds I, Ia and Ib; pharmaceutical compositions comprising such compounds; and to methods of treatment comprising administering such compounds and compositions when an antibiotic effect is indicated.

There is a continuing need for new antibiotics. For unfortunately, there is no static effectiveness of any given antibiotic because continued wide scale usage selectively gives rise to resistant strains of pathogens. In addition, the

known antibiotics suffer from the disadvantage of being effective only against certain types of microorganisms. Accordingly, the search for new antibiotics continues.

Thus, it is an object of the present invention to provide a novel class of antibiotics which are useful in animal and human therapy and in inaminate systems. These antibiotics are active against a broad range of pathogens which representatively include both Gram positive bacteria such as S. aureus, Strep. pyogenes, and B. subtilis, and Gram negative bacteria such as E. coli, Pseudomonas, Proteus morganii, Serratia, and Klebsiella. Further objects of this invention are to provide chemical processes for the preparation of such antibiotics and their nontoxic, pharmaceutically acceptable salts; pharmaceutical compositions comprising such antibiotics; and to provide methods of treatment comprising administering such antibiotics and compositions when an antibiotic effect is indicated.

DETAILED DESCRIPTION OF THE INVENTION

The compounds of the present invention (I, above) are conveniently prepared by the following scheme:

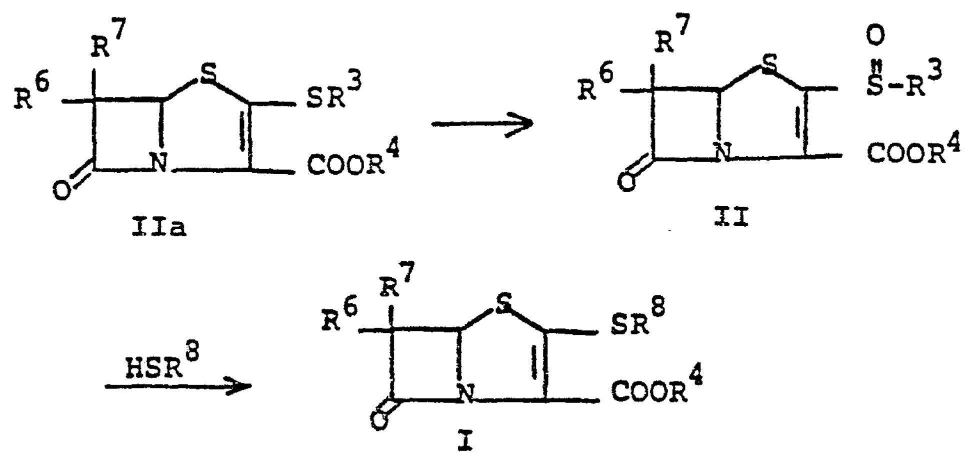


IIa

II

I

The compounds of the present invention (Ia, above) are also conveniently prepared by the same scheme:

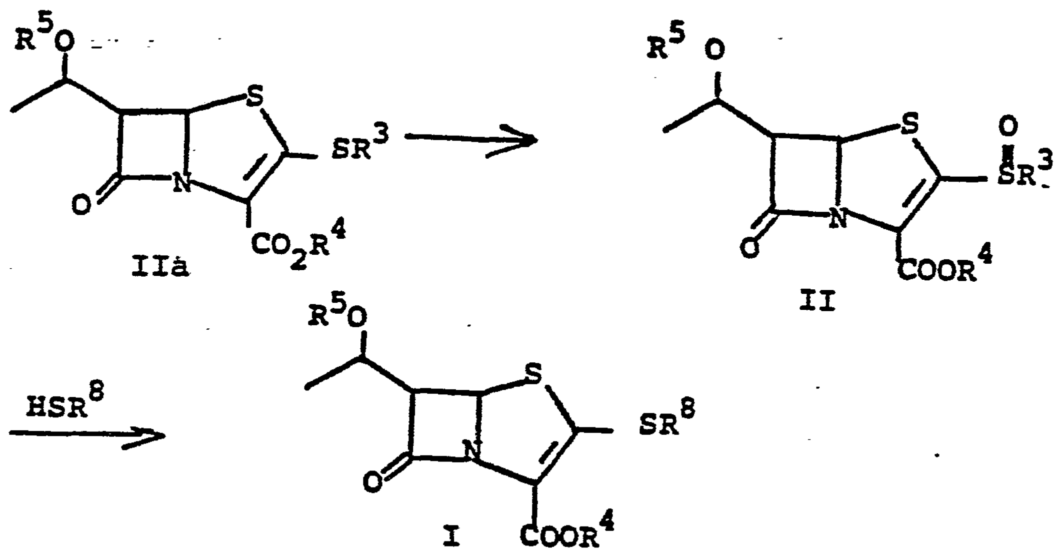


IIa II I

Transformation IIa to II.

Relative to the above reaction scheme, there is no undue criticality as to the precise identity of the oxidizing agent. Suitable oxidizing agents include peracids such as m-chloroperbenzoic acid and peracetic acid. Other representative oxidizing agents include potassium permanganate, hydrogen peroxide, sodium meta periodate, t-butyl hydrogen peroxide, dichloroiodobenzene, sulfuryl chloride with wet silica gel, sodium hypochlorite, and ozone, for example. Typically, 1.0 eq. to a slight excess of oxidizing reagent is employed. There is no criticality as to reaction solvent--any solvent being acceptable which is inert or substantially inert during the course of reaction and which effectively solubilizes the starting material IIa. Representative examples of suitable solvents for the oxidation include tetrahydrofuran, methylenechloride, dimethylformamide, methanol and water. Typically, the reaction is conducted at a temperature of from about -78 to 50°C, for from a few minutes to several hours. As mentioned above, starting materials IIa are known.

Transformation II to I

The transformation II to I is accomplished by treating II with the reagent of choice, $HSR^8$, in the presence of a base. Suitable bases for this reaction include N-heterocycles, amines, trialkylamines, and inorganic bases, such as, diisopropylethylamine, triethylamine, N-methylpiperidine, potassium carbonate, and sodium bicarbonate. In the alternative, the reaction may proceed without the addition of base when the $HSR^8$ reagent is taken as its salt: $M^{+-}SR^8$ wherein $M^+$ is $Li^+$, $Na^+$, $K^+$ or $R^4N^+$, for example: wherein R is independently chosen from: alkyl having 1-16 carbon atoms or aralkyl having 7-12 carbon atoms; for example: methyl, ethyl, butyl, benzyl, hexadecyl or the like. Typically, the reaction is run in a solvent such as dimethylformamide, acetonitrile, dimethylsulfoxide, water or mixtures thereof, at a temperature of from -50 to 30°C (preferably in the rang from -30° to 15°C) for from 1 min to 24 hours. Reactions are conducted in water as the solvent or cosolvent can be kept in the pH 7-8.5 range with a suitable buffer.

Preferably, the reaction II to I is conducted in the presence of a sulfenic acid trap. The sulfenic acid trap may be accomplished by using excess $HSR^8$ or, in the alternative, using relatively, non-reactive, mercaptans such as trityl mercaptan, olefins such as: cyclohexene, isobutylene, dihydropyran; phosphines such as triphenyl phosphine, tributylphosphine, or the like, may also be employed.

Relative to starting material II concurrently filed, commonly assigned U.S. Patent Applications Serial Numbers 353,451 (filed March 1, 1982 ) and 353,452 (filed March 1, 1982 ) [Merck & Co., Inc., Attorney's Docket Numbers 16704 and 16636 respectively] are incorporated herein by reference to the extent that they define II and its manner of preparation:

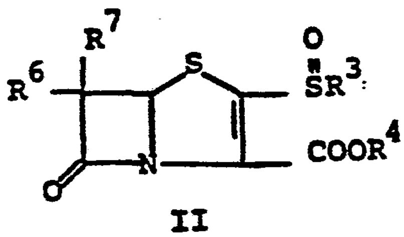

II

and

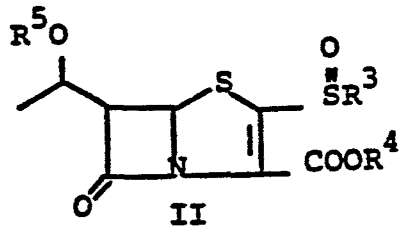

II

In brief, relative to starting material II, $R^6$, $R^7$ and $R^4$ are as defined, $R^3$ is alkyl, aralkyl or cycloalkyl, and $R^5$ is hydrogen or a hydroxyl protecting group; such hydroxyl protecting groups include: triorganosilyl wherein the organo moiety is independently selected from alkyl having 1-6 carbon atoms, phenyl, and phenylalkyl, for example, t-butyldimethylsilyl; $PNBO_2C$, $TCEO_2C$, and the like. They are prepared from the corresponding 2-$SR^3$ species (IIa) by oxidation. The 2-$SR^3$ starting penems are known. See U.S. Patent 4,260,618 (issued 4-7-81); U.K. Patent Application GB 201 3674A (published 15 August 1979); and U.K. Patent Application G.B. 2042520H (published 24 September 1980), which documents are incorporated herein by reference. [PNB = p-nitrobenzyl; TCE = 2,2,2-trichloroethyl.]

<u>$HSR^8$ REAGENTS</u>

Relative to the foregoing description of the invention, suitable carbamimidoyl and carbamimidinium mercaptans $HSR^8$ which are utilized in the transformation II to I are listed below. Wherein $R^8$ is:

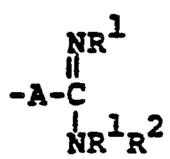

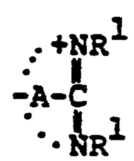

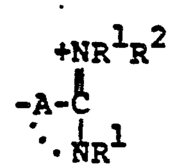

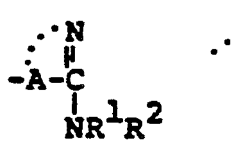

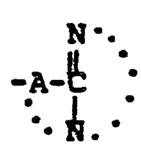

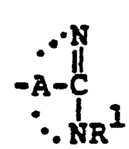

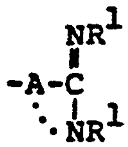 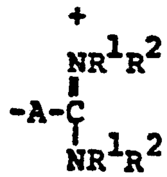 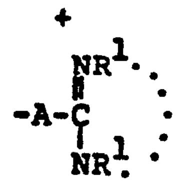

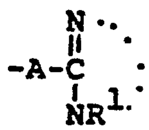 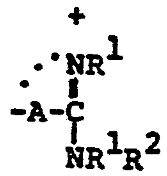 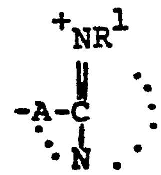

and wherein $R^1$ and $R^2$ are as initially defined under $R^8$; the two nitrogen atoms demonstrated in the above structure may participate in cyclic structures which are indicated by the dotted lines; A is a connecting group between the sulfur atom and carbamimidoyl function. It should be noted that while not all conical forms of $R^8$ are reproduced herein, the foregoing list is representative and constitutes together with the associated text a definition of the "carbamimidoyl" group of the present invention.

$R^1$ and $R^2$ are independently selected from the group consisting of hydrogen, amino, mono- and disubstituted amino (wherein the substituent is alkyl having 1-6 carbon atoms); alkoxyl (having 1-6 carbon atoms; substituted and unsubstituted: straight and branched alkyl having from 1 to 6 carbon atoms; cycloalkyl having 3 to 6 carbon atoms; cycloalkylalkyl wherein the cycloalkyl moiety comprises 3 to 6 carbon atoms and the alkyl moiety comprises 1 to 6 carbon atoms; alkylcycloalkyl wherein the alkyl moiety comprises 1 to 6 carbon atoms and the cycloalkyl moiety comprises 3 to 6 carbon atoms; aryl such as phenyl; arylalkyl such as benzyl; heterocyclyl (saturated and unsaturated) comprising mono- and bicyclic structures having from 5 to 10 ring atoms, wherein one or more of the heteroatoms is selected from oxygen, nitrogen,

or sulfur, such as thiophene, imidazole, tetrazolyl, furyl, pyridine; heterocyclylalkyl groups which comprise the immediately preceding heterocyclyl moieties and the alkyl moiety comprises 1 to 6 carbon atoms. The substituent or substituents relative to the above-named radicals comprising $R^1$ and $R^2$ are selected from the group consisting of amino, hydroxy, cyano, carboxyl, nitro, chloro, bromo, fluoro, alkoxy, and alkylthio having from 1 to 6 carbon atoms, mercapto, perhaloalkyl having 1 to 3 carbon atoms, guanidino, amidino, sulfamoyl. When located on the same nitrogen atom, the substituents $R^1$ and $R^2$ can be joined to form a cyclic group comprising 3-8 atoms. The resulting ring can contain additional O, S or N atoms. For example, $-NR^1R^2$ can be taken as morpholino, pyrrolidino, piperidino, azetidinyl or the like.

Particularly preferred groups under the definition of $R^1/R^2$ are: hydrogen; substituted and unsubstituted: straight and branched loweralkyl having from 1 to 6 carbon atoms; cycloalkyl having from 3 to 6 carbon atoms, cycloalkylalkyl wherein the cycloalkyl moiety comprises 3 to 6 carbon atoms and the alkyl moiety comprises 1 to 6 carbon atoms; aryl such as phenyl, arylalkyl such as benzyl; the substituents on the above-named radicals are selected from fluoro, hydroxy, mercapto, alkoxy and alkylthio having from 1 to 3 carbon atoms.

In defining the bivalent, cyclic or acyclic connector group "A", it is to be noted that the recited radicals of definition are to be read both left to right and right to left. Thus, the preferred connecting groups "A" are selected from: substituted

and unsubstituted: loweralkyl having from 1-6 carbon atoms; cycloalkyl having from 3-10 atoms; cycloalkylalkyl wherein the cycloalkyl moiety comprises 3 to 6 carbon atoms and the alkyl moiety comprises 1 to 10 carbon atoms; alkylcycloalkyl wherein the alkyl moiety comprises 1 to 6 carbon atoms and the cycloalkyl moiety comprises 3 to 6 carbon atoms; loweralkenyl having from 2-10 carbon atoms, cycloalkenyl having from 3 to 10 carbon atoms, cycloalkenylalkyl wherein the cycloalkenyl moiety comprises 3 to 10 carbon atoms; and the alkyl moiety comprises 1 to 6 carbon atoms; alkynyl having from 2 to 10 carbon atoms; aryl such as phenyl and naphthyl; arylalkyl and alkylaryl such as benzyl, phenethyl and the like; heteroalkyl, alkylheteroalkyl, arylheteroarlky and alkylheteroaryl wherein the hetero atoms are selected from the group of sulfur, oxygen and nitrogen, and the alkyl moiety has 1 to 6 carbon atoms, and the aryl moiety is phenyl; heterocyclyl (saturated and unsaturated) comprising mono- and bicyclic structures having 5 to 10 ring atoms wherein one or more of the hetero atoms is selected from oxygen, nitrogen, or sulphur such as thiophene, imidazole, pyridine, furyl and the like; heterocyclyalkyl wherein the heterocyclyl moiety comprises from 3 to 10 atoms and the alkyl moiety comprises from 1 to 6 atoms; the substituent (or substituents) relative to the above-named radicals are selected from the group consisting of amino, hydroxyl, cyano, carboxyl, nitro, chloro, bromo, fluoro, alkoxy having from 1 to 6 carbon atoms, mercapto, perhaloloweralkyl such as trifluoromethyl and alkylthio having from 1-6 carbon atoms.

A particularly preferred class of connecting groups "A" are selected from: a single bond connecting the sulfur and carbamimidoyl function; substituted and unsubstituted: straight and branched loweralkyl having from 1 to 6 carbon atoms, cycloalkyl having from 3 to 6 carbon atoms; phenyl; heterocyclyl such as thiophene, imidazole, pyridine, and furane; alkylheteroalkyl wherein alkyl moiety comprises 1 to 3 carbon atoms and the hetero atoms are sulfur, oxygen and nitrogen; the substituents relative to the above-named radicals are: amino, hydroxyl, chloro, bromo, fluoro, cyano, carboxyl alkoxy having from 1 to 3 carbon atoms, mercapto, trifluoromethyl, and alkylthio having from 1 to 3 carbon atoms.

Representative examples of such preferred $-SR^8$ groups (represented as $HSR^8$) are:

EXAMPLES

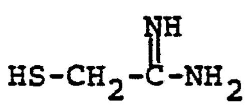

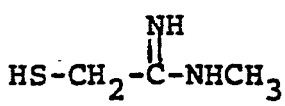

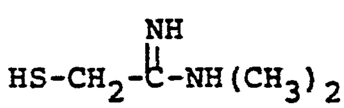

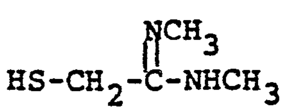

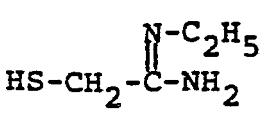

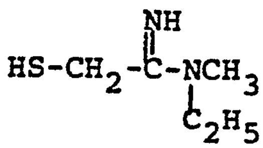

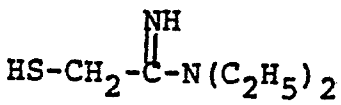

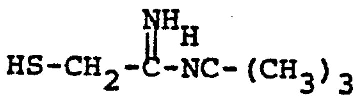

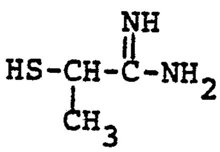

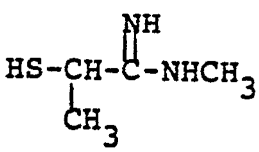

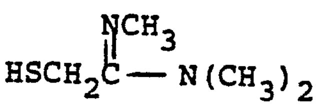

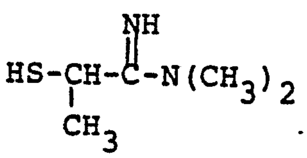

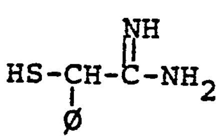

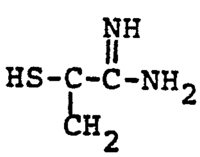

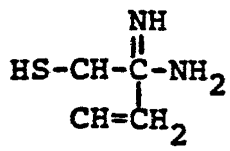

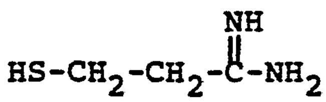

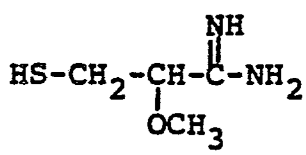

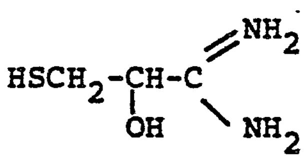

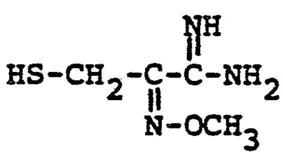

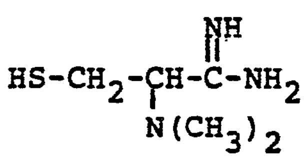

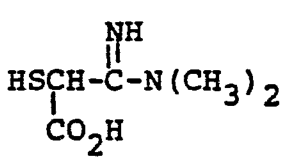

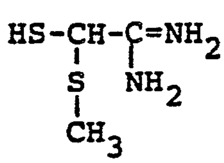

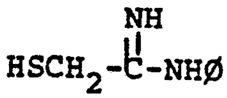

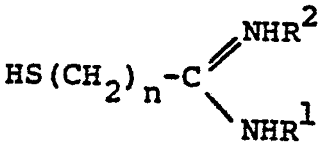

$n = 2\text{-}5$, $R^2 = H, CH_3$
$R^1 = H, CH_3$

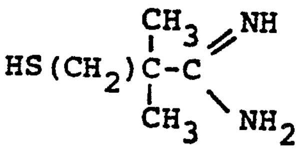

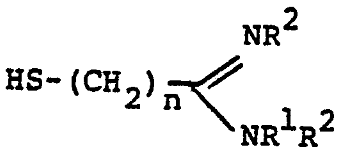

$n = 2\text{-}5$, $R^1$, $R^2 = H, CH_3$

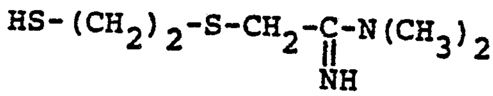

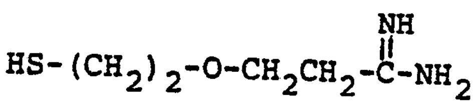

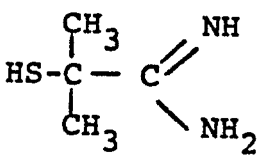

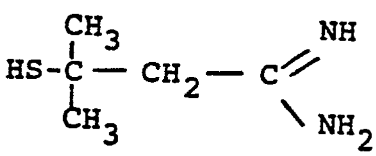

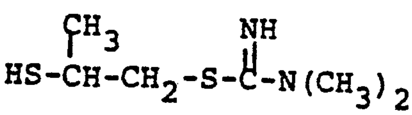

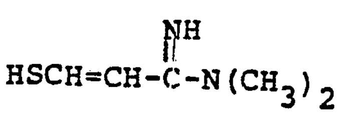

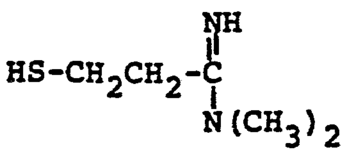

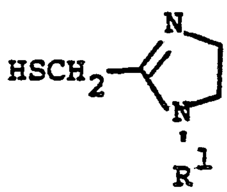

$R^1$ = H, $CH_3$

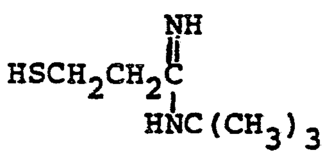

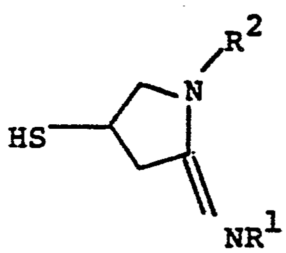

$R^1$ = H, $CH_3$
$R^2$ = H, $CH_3$

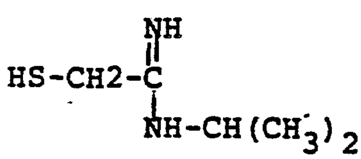

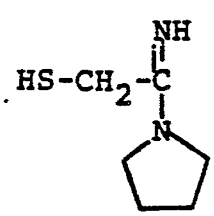

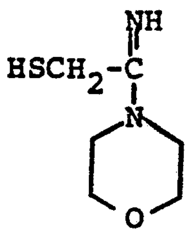

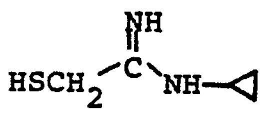

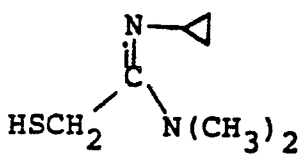

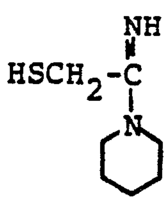

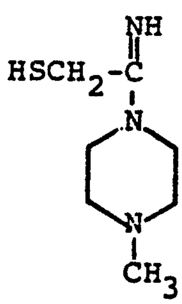

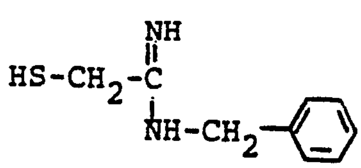

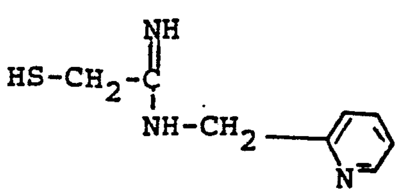

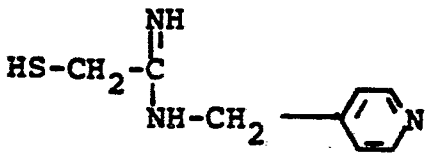

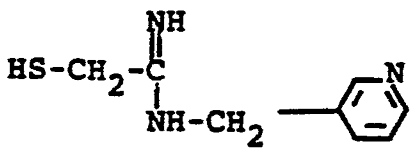

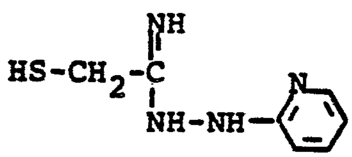

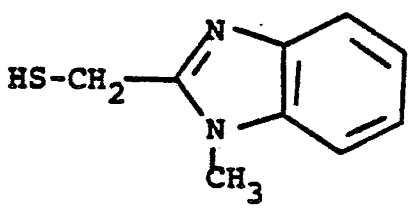

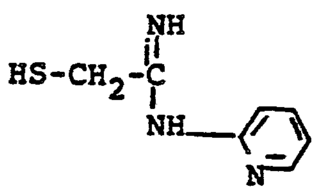

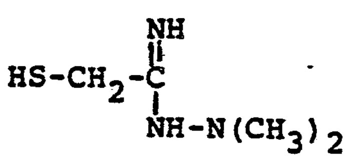

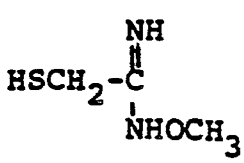

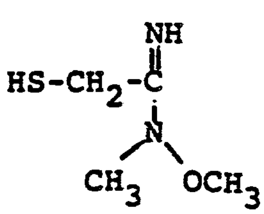

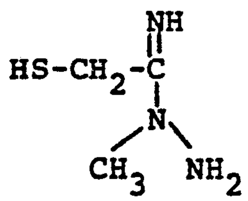

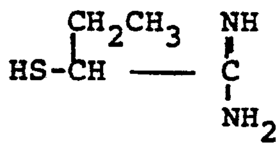

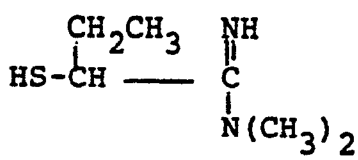

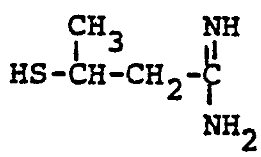

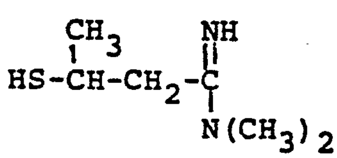

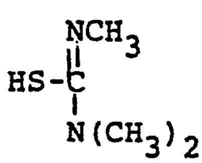

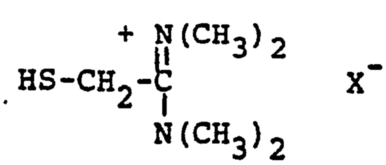

X = any compatible anion

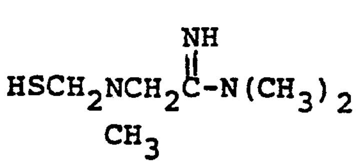

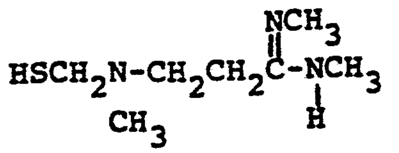

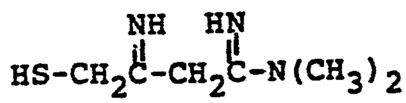

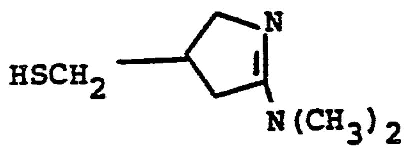

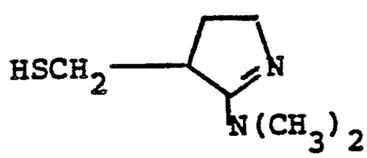

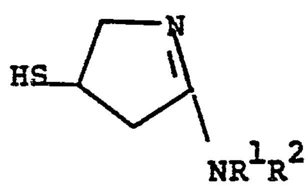

$R^1$ = H, $CH_3$
$R^2$ = H, $CH_3$

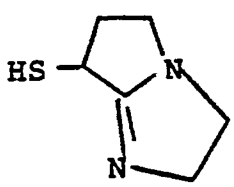

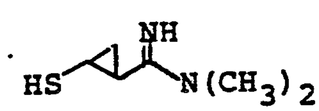

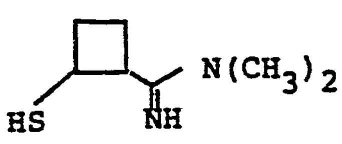

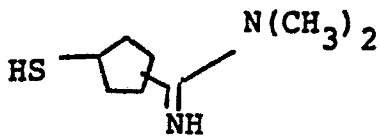

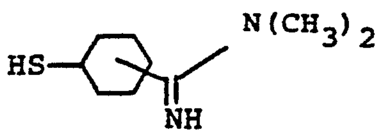

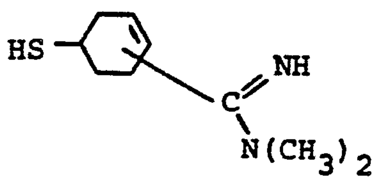

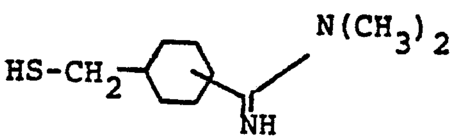

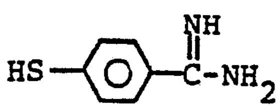

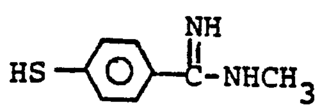

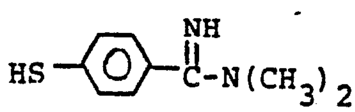

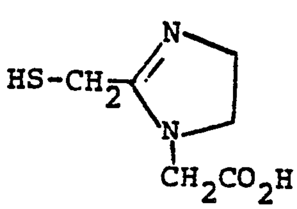

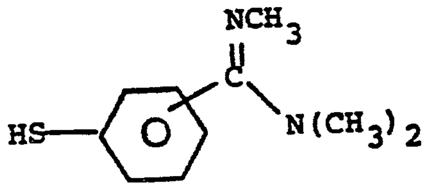

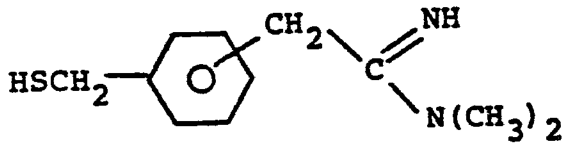

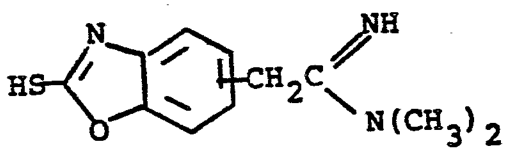

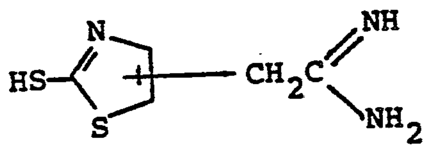

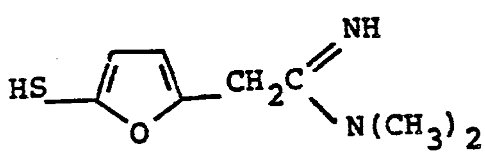

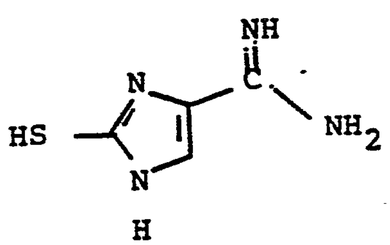

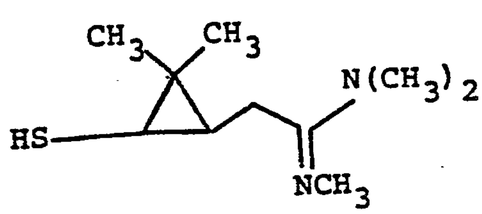

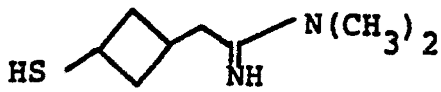

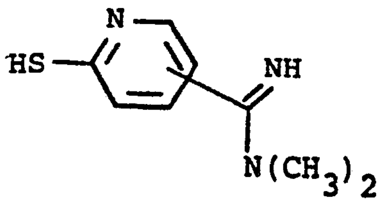

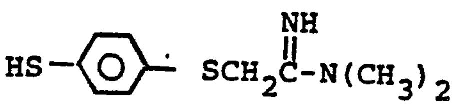

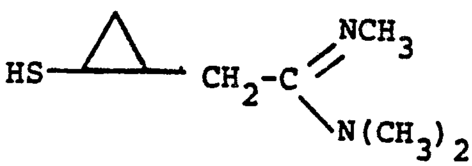

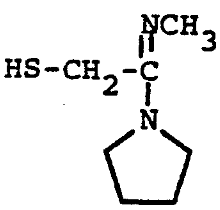

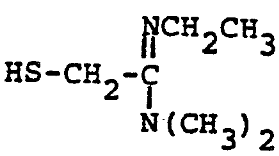

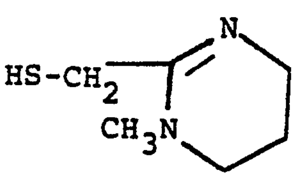

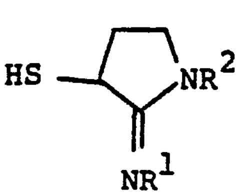

$R^1$ = H, $CH_3$

$R^2$ = H, $CH_3$

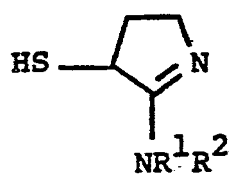

$R^1$ = H, $CH_3$
$R^2$ = H, $CH_3$

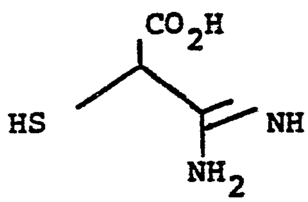

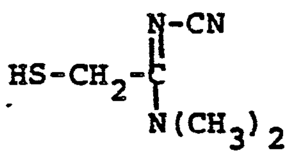

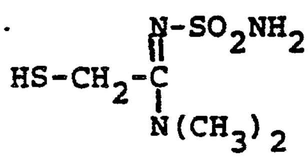

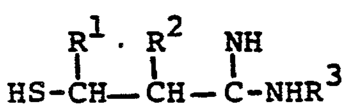

$R^1$ = $CH_3$
$R^2$ = $CH_3$, $N(CH_3)_2$, $OCH_3$
$R^3$ = H, $CH_3$

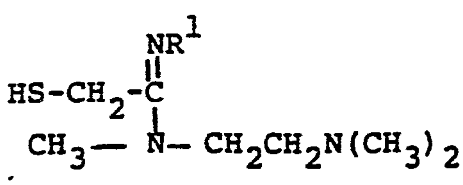

$R^1$ = H, $CH_3$

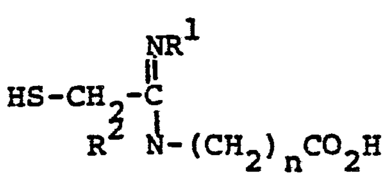

$R^1$ = H, $CH_3$
$R^2$ = H, $CH_3$
n = 1 or 2

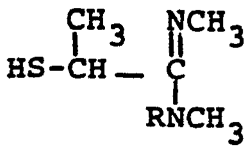

R = H, $CH_3$

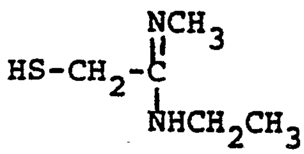

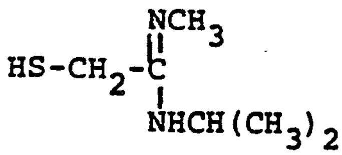

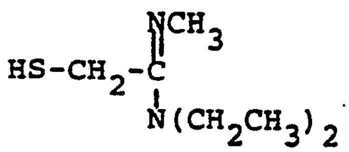

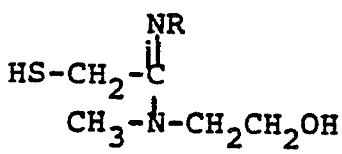

R = $CH_2CH_3$, $CH_3$, H

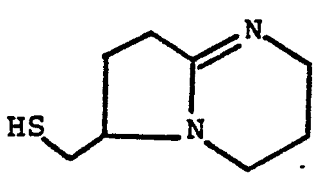

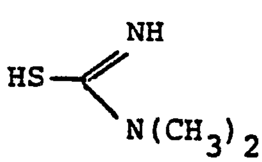

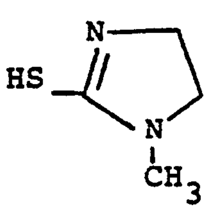

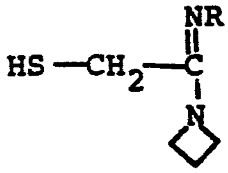

R = H, $CH_3$

It is recognized that $SR^8$ side chains in which the $R^8$ group contains one or more chiral centers can be added as racemic or diastereomeric mixtures to provide mixtures of diastereomeric products or can be added as resolved, isomerically pure reagents to provide diastereomerically pure products. Since antibacterial activity and other pharmacological properties vary among isomers, it is frequently advantageous to prepare isomerically pure products by the introduction of resolved $-SR^8$ side chains.

As noted above, the compounds of the present invention may also generally be represented by the following structural formula:

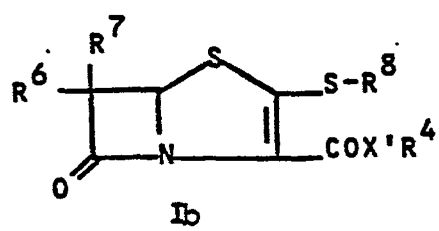

Ib

wherein X' is oxygen, sulfur or NR' (R' is hydrogen or loweralkyl having from 1 to 6 carbon atoms); and $R^4$ is hydrogen, or, <u>inter alia</u>, is representatively selected to provide the pharmaceutically acceptable salt, ester, anhydride ($R^4$ is acyl), and amide moieties known in the bicyclic β-lactam antibiotic art; $R^4$ may also be a readily removable blocking

group or a synthetic useful salt moiety. A synthetically useful salt moiety consists of a highly lipophilic carboxylate cation which imparts organic solubility to the molecule. This type of carboxylate derivative allows reactions, and particularly the displacement reaction of II to I, to be conducted in an organic solvent. Representative examples of such highly lipophilic carboxylate cations $R^4$ are ammonium salts $R^a_4N^+$ wherein $R^a$ are independently selected from 1-16 carbon alkyl groups or 7 to 10 carbon aralkyl groups. A particularly useful example of this type is the N,N-dimethyl-N-benzyl-N-hexadecyl ammonium salt.

Identification of the Radical $-COX'R^4$

In the generic representation of the compounds of the present invention (I, above), the radical represented by $-COX'R^4$ is, inter alia, -COOH (X' is oxygen and $R^4$ is hydrogen) and all radicals known to be effective as pharmaceutically acceptable ester, anhydride ($R^4$ is acyl) and amide radicals in the bicyclic β-lactam antibiotic art, such as the cephalosporins and penicillins and nuclear analogues thereof.

Suitable, but representative, blocking esters $R^4$ (X'= O) include those selected from the following list which is representative:

(i) $R^4 = CR^aR^bR^c$ wherein at least one of $R^a$, $R^b$, and $R^c$ is an electron-donor, e.g., p-methoxyphenyl. The remaining $R^a$, $R^b$ and $R^c$ groups may be hydrogen or organic substituting groups. Suitable ester groups of this type include p-methoxybenzyloxycarbonyl.

(ii) $R^4 = CR^aR^bR^c$ wherein at least one of $R^a$, $R^b$ and $R^c$ is an electron-attracting group, e.g., p-nitrophenyl, trichloromethyl, and o-nitrophenyl. Suitable esters of this type include p-nitrobenzyloxycarbonyl, 2,2,2-trichloroethoxycarbonyl, and acetonyloxycarbonyl.

(iii) $R^4 = CR^aR^bR^c$ wherein at least two of $R^a$, $R^b$ and $R^c$ are hydrocarbon such as alkyl, e.g., methyl or ethyl, or aryl, e.g., phenyl and the remaining $R^a$, $R^b$ and $R^c$ group, if there is one, is hydrogen. Suitable esters of this type include t-butyloxycarbonyl, diphenylmethoxycarbonyl, triphenylmethoxycarbonyl, and allyloxycarbonyl.

Silyl esters. This category of blocking groups, may conveniently be prepared from a halosilane of the formula: $R_3^{4°}SiX°$ wherein X° is a halogen such as chloro or bromo and $R^{4'}$ is independently chosen from: alkyl, having 1-6 carbon atoms, phenyl, or phenylalkyl. Suitable esters of this type include t-butyldiphenylsilylcarbonyl.

Pharmaceutically acceptable carboxyl derivatives of the present invention are those derived by reacting I with alcohols, acylating reagents and the like. For example, esters and amides of interest are the above-listed starting materials and final products having the $-COX'R^4$ group at the 3-position; wherein X' is oxygen, sulfur or NR' (R' is H or $R^4$), and $R^4$ is alkyl having 1-6 carbon atoms, straight or branched, such as methyl, ethyl, t-butyl, and the like; carbonylmethyl, including phenacyl; aminoalkyl including 2-methylaminoethyl, 2-diethylaminoethyl; alkanoyloxyalkyl wherein the alkanoyloxy portion is straight or branched and has 1-6 carbon atoms and the

alkyl portion has 1-6 carbon atoms, such as pivaloyloxymethyl; haloalkyl wherein halo is chloro, and the alkyl portion is straight or branched having 1-6 carbon atoms, e.g., 2,2,2-trichloroethyl; alkenyl having 1-4 carbon atoms, such as 2-propenyl, 3-butenyl, and 4-butenyl; aralkyl and lower alkoxyl- and nitro- substituted aralkyl such as benzyl, benzhydryl, o-nitrobenzyl, p-methoxybenzyl, and p-nitrobenzyl; phthalidyl; benzyloxyalkyl having 8-10 carbon atoms, such as benzyloxymethyl, and (4-nitro) benzyloxymethyl.

In addition to the esters (and thio esters) listed above, amides are also embraced by the present invention, i.e., wherein X' is the -NR'- group. Representative of such amides are those wherein R' is selected from the group consisting of hydrogen and alkyl such as methyl and ethyl.

The most preferred $-COX'R^4$ radicals of the present invention are those wherein (relative to Structure I above), X' is oxygen and $R^4$ is hydrogen; loweralkyl having 1-4 carbon atoms; lower alkenyl such as 3-methylbutenyl, allyl, 2,2,2-trichloroethyl, 4-butenyl and the like; benzyl and substituted benzyl such as o-nitrobenzyl, p-nitrobenzyl; pivaloyloxymethyl, 3-phthalidyl; phenacyl, acetonyl; and triorganosilyl, such as trimethylsilyl.

Removal of the preferred protecting groups is categorized as follows: a.) for hydroxyl and amino functionalities bearing p-nitrobenzyloxycarbonyl protection or a carboxyl functionality possessing a p-nitrobenzyl moiety deprotection is accomplished by catalytic hydrogenation over a transition metal catalyst such as palladium supported on carbon,

palladium hydroxide on carbon, or plantinum oxide, in an inert solvent or solvent mixtures, which maybe buffered in the usual way, such as tetrahydrofuran, dioxane, ethyl acetate, ethanol, and water, at a temperature of from 0°C to ambient temperature, at a pressure of from 1 atmosphere to 5 atomospheres of hydrogen, for a period of from a few minutes to twelve hours; b.) for hydroxyl functions covered by a tbutyldimethylsilyl (TBDMS) group removal is accomplished according to the procedure of G. Just and T. J. Liak, Can. J. Chem., 56, 211 (1978), which comprises treating the TBDMS ether derivative with N-tetrabutyl ammonium fluoride in the presence of acetic acid in an inert solvent such as tetrahydrofuran at a temperature of from -78°C to ambient temperature for from a few minutes to 72 hours; c.) for carboxyl moieties possessing an allyl moiety and for hydroxyl and amino groups bearing an allyloxycarbonyl function deprotection is accomplished by treatment with a combination of triphenyl phosphine, tetrakistriphenylphosphine palladium (O), and 2-ethylhexanoic acid or its sodium or potassium salts in a suitable aprotic solvent such as ethylacetate, methylene chloride, tetrahydrofuran, or diethylether. Use of either sodium or potassium 2-ethylhexanoate provides the corresponding salt; whereas 2-ethylhexanoic acid provides the free carboxylic acid. The process for removing an allylic group from allylic esters, carbonates and carbamates is described in European Patent Application 13,633 (Schering Corp.) and in S.W. McCombie, et al., Tetrahedron Letters, 22, 3489 (1981).

PREFERRED VALUES FOR $R^6$ and $R^7$

In the generic structure (I):

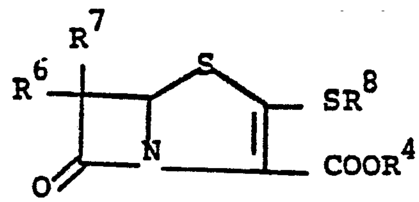

The preferred values for $R^6$ and $R^7$ independently are selected from:

H
$CH_3CH_2$
$CH_3$
$CH_3CH_2CH(OH)$
$FCH_2CH(OH)$
$CF_3CH(OH)$
$CH_2OH$
$(CH_3)_2C(OH)$
$CH_3CF_2$
ØCH$_2$ [Ø = phenyl]
$(CH_3)_2CHCH(OH)$
$CH_3CH(NH_2)$
$CH_3CH(OH)$
$HOCH_2CH_2$
$CH_3O$
$CH_3CH[NH(CH_3)]$
$(CH_3)_2CH$
$CH_3CH_2CH_2CH(OH)$
$HOCH_2CH(OH)$
$CH_3O_2C$
$NH_2OC$
$CH_3NH(CO)$
$CH_2=CHCH(OH)$

$HO_2CCH_2$

$CF_3$

$CH_2=CH$

$HC\equiv C$

$H_2NCH_2$

$HCF_2CH_2$

$CH_2=$

$(HOCH_2)(CH_3)C=$

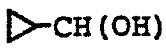

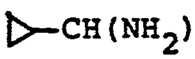

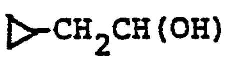

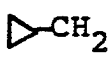

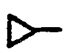

$CH_3O_2CCH_2$

$CH_3OCH_2CH(OH)$

$ClCH_2CH(OH)$

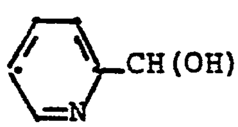

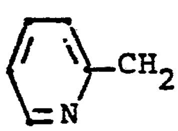

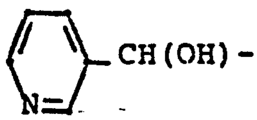

An especially preferred substitution at position 6 finds $R^6$=H, and $R^7$ selected from: hydrogen, $FCH_2CH(OH)-$, $CH_3CH_2CH(OH)-$, $CH_3CH_2-$, $OCH_3$, $(CH_3)_2CH-$, 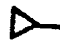$CH(OH)-$, $(CH_3)_2C(OH)-$, and $HOCH_2$; when $R^6$ is not hydrogen, $R^7$ is as designated in this sentence and additionally includes $CH_3CH(OH)-$.

More especially preferred are compounds of the present invention, Ia:

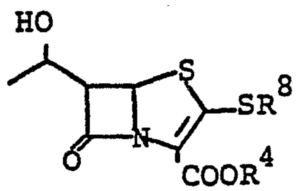

wherein: $R^4$ and the preferred $-SR^8$ groups are as defined above.

Relative to the generic description of the compounds of the present invention, I:

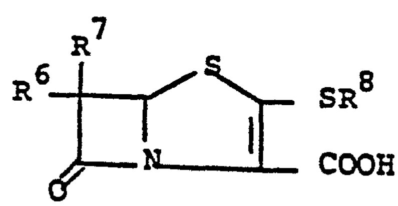

the following representative drawings depict the most preferred configurations:

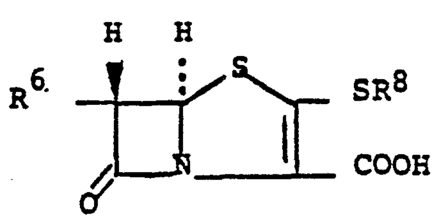

$R^6 = CH_3CH_2$, $(CH_3)_2CH$, $CH_3O$, $HOCH_2$, for example.

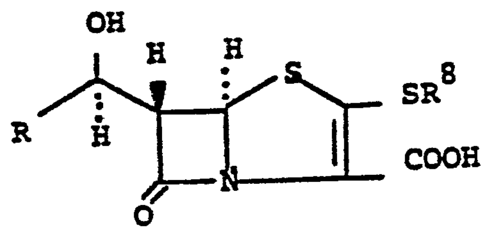

R = $FCH_2$, $CH_3CH_2$, for example.

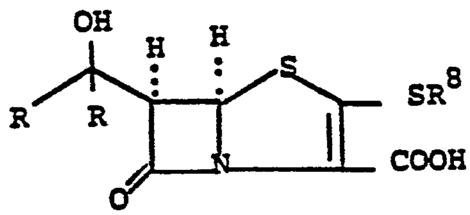

R = $CH_3$, $-CH_2-$, for example.

Relative to the generic description of the compounds of the present invention, Ic:

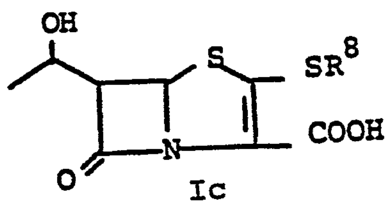

Ic

the following drawing depicts the most preferred configuration:

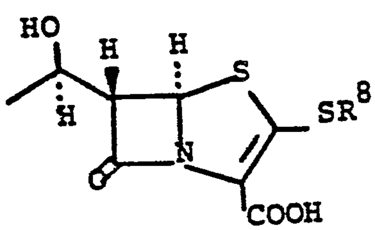

The compounds of the present invention are valuable antibiotics active against various Gram-positive and Gram-negative bacteria and accordingly find utility in human and veterinary medicine. Representative pathogens which are sensitive to antibiotics I include: Staphylococcus aureus, Escherichia coli, Klebsiella pneumoniae, Bacillus subtilis, Salmonella typhosa, Psuedomonas and Bacterium proteus. The antibacterials of the invention are not limited to utility as medicaments; they may be used in all manner of industry, for example: additives to animal feed, preservation of food, disinfectants, and in other industrial systems where control of bacterial growth is desired. For example, they may be employed in aqueous compositions in concentrations ranging from 0.1 to 100 parts of antibiotic per million parts of solution in order to destroy or inhibit the growth of harmful bacteria on medical and dental equipment and as bactericides in industrial applications, for example in waterbased paints and in the white water of paper mills to inhibit the growth of harmful bacteria.

The products of this invention may be used in any of a variety of pharmaceutical preparations. They may be employed in capsule, powder form, in

liquid solution, or in suspension. They may be administered by a variety of means; those of principal interest include: orally, topically or parenterally by injection (intravenously or intramuscularly).

Such tablets and capsules, designed for oral administration, may be in unit dosage form, and may contain conventional excipients, such as binding agents, for example, syrup, acacia, gelatin, sorbitol, tragacanth, or polyvinylpyrrolidone; fillers, for example, lactose, sugar, cornstarch, calcium phosphate, sorbitol, or glycerine; lubricants, for example, magnesium stearate, talc, polyethylene glycol, silica; disintegrants, for example, potato starch, acceptable wetting agents such as sodium lauryl sulphate. The tablets may be coated according to methods well known in the art. Oral liquid preparations may be in the form of aqueous or oily suspensions, or solutions, or they may be presented as a dry product for reconstitution with water or other suitable vehicle before use. Such liquid preparations may contain conventional additives such as suspending agents, for example, sorbitol, methyl cellulose, glucose/sugar syrup, gelatin, hydroxyethylcellulose, or carboxymethyl cellulose. Suppositories will contain conventional suppository bases, such as cocoa butter or other glycerides.

Compositions for injection, the preferred route of delivery, may be prepared in unit dosage form in ampules, or in multidose containers. The compositions may take such forms as suspensions, solutions, or emulsions in oily or aqueous vehicles, and may contain formulatory agents. Alternatively,

the active ingredient may be in powder form for reconstitution, at the time of delivery, with a suitable vehicle, such as sterile water.

The compositions may also be prepared in suitable forms for absorption through the mucous membranes of the nose and throat or bronchial tissues and may conveniently take the form of liquid sprays or inhalants, lozenges, or throat paints. For medication of the eyes or ears, the preparation may be presented in liquid or semi-solid form. Topical applications may be formulated in hydrophobic or hydrophilic bases as ointments, creams, lotions, paints, or powders.

The dosage to be administered depends to a large extent upon the condition and size of the subject being treated as well as the route and frequency of administration -- the parenteral route by injection being preferred for generalized infections. Such matters, however, are left to the routine discretion of the therapist according to principles of treatment well known in the antibiotic art. In general, a daily dosage consists of from about 5 to about 600 mg of active ingredient per kg. of body weight of the subject in one or more treatments per day. A preferred daily dosage for adult humans lies in the range of from about 10 to 240 mg. of active ingredient per kg. of body weight. Another factor influencing the precise dosage regimen, apart from the nature of the infection and peculiar identity of the individual being treated, is the molecular weight of the chosen species of this invention (I).

The compositions for human delivery per unit dosage, whether liquid or solid, may contain from

0.1% to 99% of active material, the preferred range being from about 10-60%. The composition will generally contain from about 15 mg. to about 1500 mg. of the active ingredient; however, in general, it is preferable to employ a dosage amount in the range of from about 250 mg to 1000 mg. In parenteral administration, the unit dosage is usually the pure compound I in sterile water solution or in the form of a soluble powder intended for solution.

In the foregoing word description, it is to be understood that there is considerable latitude in selection of precise reaction parameters. Suggestion of this latitude and its breadth is generally indicated by the enumeration of equivalent solvent systems, temperature ranges, protecting groups, and range of identities of involved reagents.

The following examples recite a precise scheme of synthesis. It is to be understood that the purpose of this recitation is to further illustrate the invention and not to impose any limitation. Temperature is in °C.

EXAMPLE 1

Preparation of

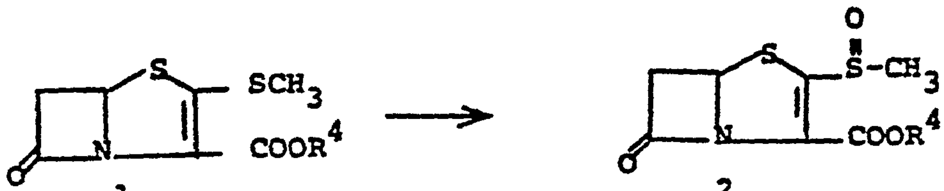

1 2

$R^4$ = p-nitrobenzyl

To a stirred solution of 11.3 mg. (0.032 mmol) of penem 1 in 1 ml. of methylene chloride at 0°C. in an ice-$H_2O$ bath is added 7.2 mg. (0.035 mmol) of 85% m-chloroperbenzoic acid. The mixture is stirred at 0°C under an atmosphere of nitrogen for 1.0 hour after which time an excess of basic resin (Amberlyst-21) is added and stirring continued for 5 minutes. The resin is removed by filtration and the filtrate is purified directly by plate layer chromatography (PLC) using $CH_2Cl_2$-EtOAc (1:1) as the eluant, to provide 6.5 mg. (55%) of sulfoxides 2 as a white foam; IR($CH_2Cl_2$) 1790, 1720, 1690 $cm^{-1}$; 300 MHZ NMR ($CDCl_3$) $\delta$: 2.94, 2.98 (s, 3H), 3.74, 3.80 (dd's, J=2, 18, Hz, 1H), 3.94, 4.0 (dd, J=4, 18 Hz, 1H), 5.3 (d's, J=14 Hz, 1H), 5.46 (d's, J=14 Hz, 1H), 5.82, 5.96 (dd's, J=2,4Hz, 1H), 7.64 (d's, J=9Hz, 2H), 8.3 (d, J=9Hz, 2H).

EXAMPLE 2

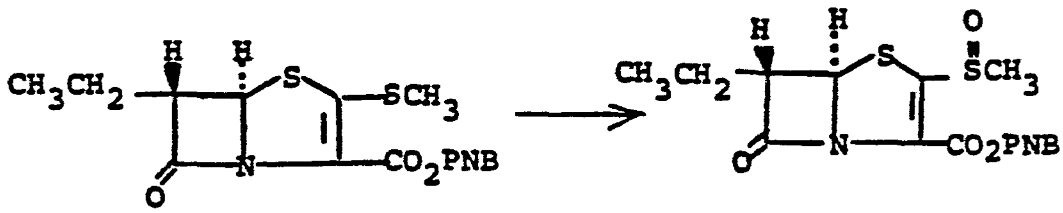

p-Nitrobenzyl (5R,6S)-2-methylsulfinyl-6-ethyl-pen-2-em-3-carboxylate

A solution of 85% m-chloroperoxy benzoic acid (53 mg, 0.26 mmol) in $CH_2Cl_2$ (1 ml) is added dropwise over a few minutes to an ice-cold, stirring solution of p-nitrobenzyl (5R,6S)-2-methylthio-6-ethyl-pen-2-em-3-carboxylate (94 mg, 0.25 mmol) in anhydrous THF (2 ml). The resulting solution is stirred an additional 30 minutes at 0°, then diluted with EtOAc and washed with $H_2O$, 5% aqueous $NaHCO_3$, $H_2O$, and brine. The organic phase is dried with $MgSO_4$, filtered, and evaporated in vacuo to provide the title compound as a mixture of sulfoxide isomers.

EXAMPLE 3

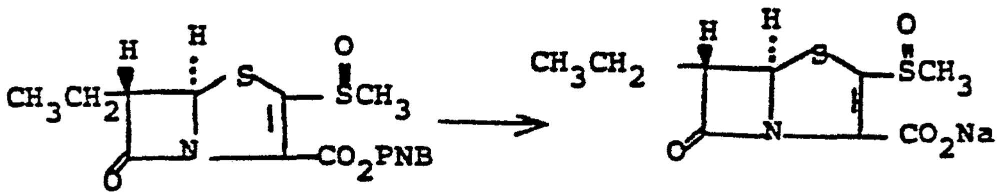

Sodium (5R, 6S)-2-methylsulfinyl-6-ethyl-pen-2-em-3-carboxylate

A solution of p-nitrobenzyl (5R,6S)-2-methylsulfinyl-6-ethyl-pen-2-em-3-carboxylate (50 mg) in THF (9 ml) is diluted with EtOH (4.5 ml) and $H_2O$ (7.0 ml) containing $NaHCO_3$ (10.6 mg). The resulting solution is added to a prereduced mixture of 10% Pd/C (100 mg) in EtOH (4.5 ml) and the resulting mixture is rapidly stirred under a $H_2$ atmosphere for 2 hours. The mixture is filtered to remove the catalyst which is washed with $H_2O$ (2 x 25 ml). The combined filtrate is extracted with $Et_2O$ (2 x 50 ml) concentrated under vacuum to ca. 10 ml volume, and lyophilized to yield the title compound.

EXAMPLE 4

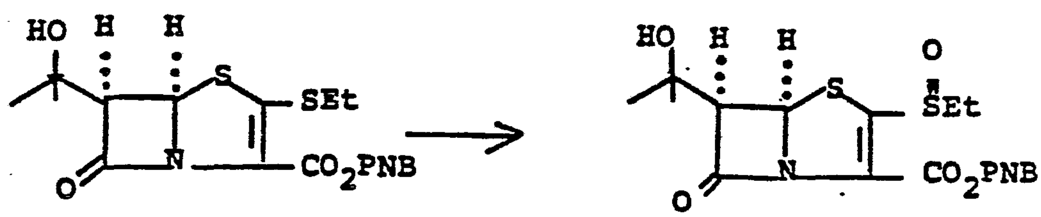

p-Nitrobenzyl (5R,6R)-2-ethylsulfinyl-6-(2-hydroxy-2-propyl)-pen-2-em-3-carboxylate

A solution of 85% m-chloroperoxybenzoic acid (112 mg, 0.55 mmol) in $CH_2Cl_2$ (2.5 ml) is added dropwise over 5 mins. to an ice-cold, stirring solution of p-nitrobenzyl (5R,6R)-2-ethylthio-6-(2-hydroxy-2-propyl)-pen-2-em-3-carboxylate (212 mg, 0.5 mmol) in $CH_2Cl_2$ (5 ml). The mixture is stirred for 1 hour at 0°, then treated with excess Amberlyst-21 resin and stirred 5 minutes longer. The mixture is filtered and the filtrate is evaporated under vacuum to provide the crude product.

EXAMPLE 5

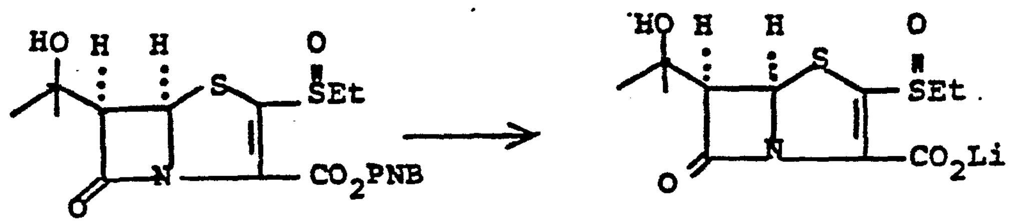

Lithium (5R,6R)-2-ethylsulfinyl-6-(2-hydroxy-2-propyl)-pen-2-em-3-carboxylate

A solution of p-nitrobenzyl (5R,6R)-2-ethylsulfinyl-6-(2-hydroxy-2-propyl)-pen-2-em-3-carboxylate (215 mg) in EtOAc (25 ml) is diluted with 1M aqueous $NaHCO_3$ solution (25 ml) and treated with 10% Pd/C (400 mg). The resulting mixture is stirred under an atmosphere of $H_2$ for 1 hour and then filtered to remove the catalyst which is washed with more EtOAc and 1M $NaHCO_3$. The aqueous portion is separated, acidified with 5% aqueous citric acid, and thoroughly extracted with $CH_2Cl_2$. The organic extracts are briefly dried over $Na_2SO_4$ and concentrated under vacuum. The residue of (5R,6R)-2-ethylsulfinyl-6-(2-hydroxy-2-propyl)-pen-2-em-3-carboxylic acid is taken up in EtOAc (20 ml) and thoroughly extracted with 0.02M LiOH solution (25 ml). The aqueous phase is separated, and lyophilized to yield the title compound.

EXAMPLE 6

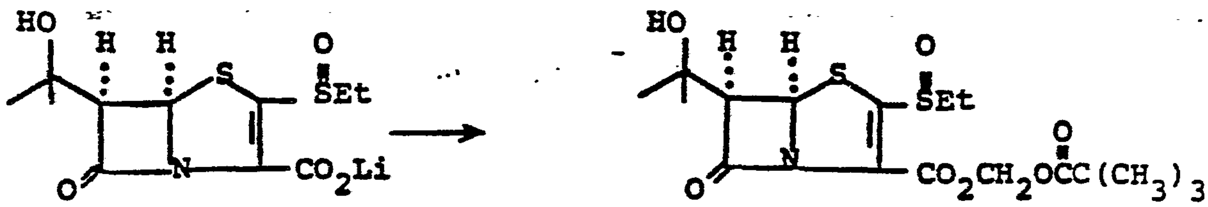

Pivaloyloxymethyl (5R, 6R)-2-ethylsulfinyl-6-(-2-hydroxy-2-propyl)-pen-2-em-3-carboxylate

Pivaloyloxymethyl bromide (29.3 mg, 0.15 mmol) is added to a suspension of lithium (5R, 6R)-2-ethylsulfinyl-6-(2-hydroxy-2-propyl)-pen-2-em-3-carboxylate (29.5 mg, 0.1 mmol) in anhydrous DMF (0.5 ml). The resulting mixture is stirred under a $N_2$ atmosphere at room temperature for 4 hours. The mixture is diluted with EtOAc, washed repeatedly with water, dried with $MgSO_4$, filtered, and evaporated under vacuum to afford a residue of the title compound. This material is purified by preparative TLC.

EXAMPLE 7

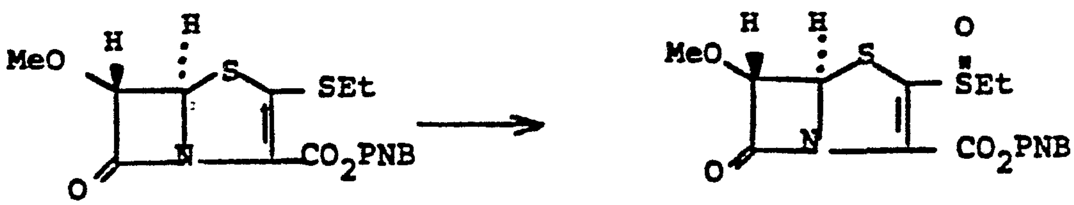

p-Nitrobenzyl (5R, 6S)-2-ethylsulfinyl-6-methoxy-pen-2-em-3-carboxylate

A solution of p-nitrobenzyl (5R, 6S)-2-ethylthio-6-methoxy-pen-2-em-3-carboxylate (96 mg, 0.24 mmol) in anhydrous DMF (1.2 ml) is diluted with $CH_2Cl_2$ (2.4 ml) and cooled in an ice-bath. A solution of 85% m-chloroperoxybenzoic acid (51 mg, 0.25 mmol) in $CH_2Cl_2$ (1.2 ml) is added dropwise over 2 minutes to the cold, stirring solution. The resulting mixture is kept at 0° for 60 minutes, and then treated with excess Amberlyst-21 resin and stirred and additional 5 minutes. The mixture is filtered and the filtrate is diluted with $CH_2Cl_2$ and washed with 5 portions of $H_2O$. The organic phase is dried over $MgSO_4$, filtered, and evaporated under vacuum to give a crude residue of the title compound which is purified by rapid chromatography on silica gel.

EXAMPLE 8

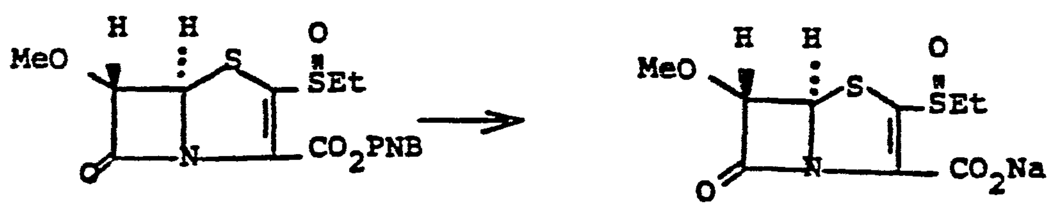

Sodium (5R, 6S)-2-ethylsulfinyl-6-methoxy-pen-2-em-3-carboxylate

A mixture of p-nitrobenzyl (5R, 6S)-2-ethylsulfinyl-6-methoxy-pen-2-em-3-carboxylate (48 mg, 0.12 mmol) and 10% pd/C (100 mg) in dioxane (8 ml) and $H_2O$ (4 ml) is shaken under a $H_2$ pressure of 45 psi for 60 minutes. The reaction mixture is diluted with $H_2O$ (20 ml) containing $NaHCO_3$ (10 mg, 0.12 mmol) and filtered to remove the catalyst. The filtrate is washed with $Et_2O$ (3 x 20 ml), concentrated under vacuum to ca. 5 ml volume, and lyophilized to provide the crude product.

## EXAMPLE 9

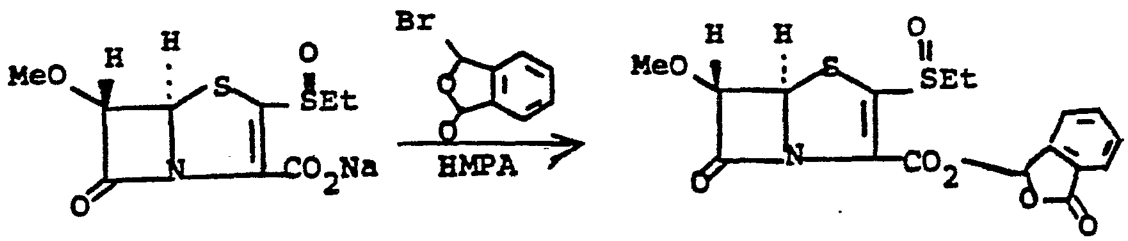


Phthalidyl (5R, 6S)-2-ethylsulfinyl-6-methoxy-pen-2-em-3-carboxylate

A mixture of sodium (5R, 6S)-2-ethylsulfinyl-6-methoxy-pen-em-3-carboxylate (28 mg, 0.1 mmol) and phthalidyl bromide (32 mg, 0.15 mmol) in anhydrous hexamethylphosphoramide (0.5 ml) is stirred at room temperature and under a $N_2$ atmosphere for 2 hours. The mixture is diluted with $Et_2O$, washed with several portions of $H_2O$, dried with $MgSO_4$, filtered, and evaporated under vacuum. The residue is purified by preparative silica gel chromatography to provide the title compound.

## EXAMPLE 10

Preparation of:

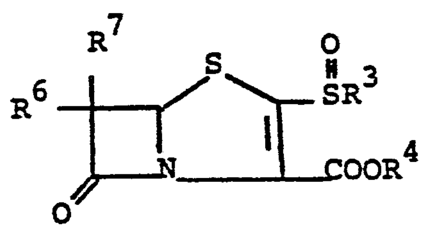

Following the procedures described in the foregoing Examples and text, the compounds listed below are obtained by analogy. PNB=p-nitrobenzyl.

| Compound | $\underline{R}^6$ | $\underline{R}^7$ | $\underline{R}^3$ | $\underline{R}^4$ | Remarks |
|---|---|---|---|---|---|
| 1 | H | $CH_3CH_2$ | $CH_3$ | $CH_2O\overset{O}{\overset{\|}{C}}CH_3$ | Obtained by alkylating $R^4$= Na compound |
| 2 | H | $CH_3CH_2$ | $CH_3$ | $CH_2O\overset{O}{\overset{\|}{C}}C(CH_3)_3$ | " |
| 3 | H | $CH_3CH_2$ | $CH_3$ | phthalidyl | " |
| 4 | H | $CH_3CH_2$ | $CH_3$ | 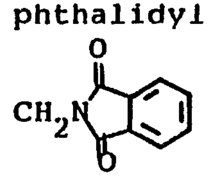 | " |
| 5 | H | $CH_3CH_2$ | $CH_3$ | $CH_2CH{=}C(CH_3)_2$ | " |
| 6 | H | $CH_3CH_2$ | $CH_3$ | 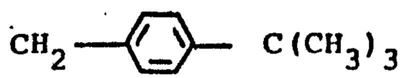 | " |
| 7 | H | $CH_3CH_2$ | $CH_3$ | 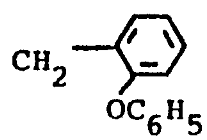 | " |

| Compound | $\underline{R}^6$ | $\underline{R}^7$ | $\underline{R}^3$ | $\underline{R}^4$ | Remarks |
|---|---|---|---|---|---|
| 8 | H | $CH_3CH_2$ | $CH_3$ | $CH(CH_3)OCO_2C_2H_5$ | " |
| 9 | H | $CH_3CH_2$ | $CH_3CH_2$ | PNB, H, Na | |
| 10 | H | $CH_3CH_2$ | $CH_2C_6H_5$ | PNB, H, Na | |
| 11 | H | $HOCH_2$ | $CH_3$ | PNB, H, Na | Hydroxyl group protected by $PNBO_2C$ derivative. Cleavage occurs in ester deblocking stage. |
| 12 | H | $HOCH_2$ | $CH_2CH_3$ | PNB, H, Na | " |
| 13 | H | $HOCH_2$ | $CH_2CH_3$ | $CH(CH_3)OCO_2C_2H_5$ | Obtained by alkylating $R^4$=Na. |
| 14 | H | $HOCH_2$ | $CH_2CH_3$ | $CH_2OCOC(CH_3)_3$ | " |
| 15 | H | $HOCH_2$ | $CH_2CH_3$ | phthalidyl | " |

| Compound | $\underline{R}^6$ | $\underline{R}^7$ | $\underline{R}^3$ | $\underline{R}^4$ | Remarks |
|---|---|---|---|---|---|
| 16 | H | $(CH_3)_2\overset{OH}{C}$ | $CH_2CH_3$ | PNB, H, Na | |
| 17 | H | $(CH_3)_2\overset{OH}{C}$ | $CH_2CH_3$ | $CH_2OCOC(CH_3)_2$ | |
| 18 | H | $(CH_3)_2\overset{OH}{C}$ | $CH_2CH_3$ | phthalidyl | |
| 19 | $OCH_3$ | $CH_3\overset{OH}{C}H$ | $CH_2CH_3$ | PNB, H, Na | Hydroxyl group protected as the $PNBO_2C$ derivative. Deblocking occurs at ester removal stage. |
| 20 | $CF_3$ | $CH_3\overset{OH}{C}H$ | $CH_2CH_3$ | PNB, H, Na | |

| Compound | $R^6$ | $R^7$ | $R^3$ | $R^4$ | Remarks |
| --- | --- | --- | --- | --- | --- |
| 21 | H | $CH_3O$ | $CH_2CH_3$ | PNB, H, Na | |
| 22 | H | $CH_3O$ | $CH_2CH_3$ | $CH_2CH=C(CH_3)_2$ | Via alkylation of $R_4$=Na intermediate |
| 23 | H | $CH_3O$ | $CH_2CH_3$ | $CH_2OCOC(CH_3)_3$ | " |
| 24 | H | $CH_3O$ | $CH_2CH_3$ | phthalidyl | " |
| 25 | H | $CH_3O$ | $CH_2CH_3$ | $CH(CH_3)OCO_2CH_2CH_3$ | " |
| 26 | H | $CH_3O$ | $CH_3$ | PNB, H, Na | |

| Compound | $R^6$ | $R^7$ | $R^3$ | $R^4$ | Remarks |
|---|---|---|---|---|---|
| 27 | H | $FCH_2\overset{\overset{OH}{\vert}}{C}H$ | $CH_2CH_3$ | PNB, H, Na | Hydroxyl group protected as $PNBO_2C$ derivative. Simultaneously cleaved in ester deblocking stage. |
| 28 | H | $CH_3\overset{\overset{NH_2}{\vert}}{C}H$ | $CH_2CH_3$ | PNB, H | Amino group protected as $PNBO_2C$ derivative. Cleaved in ester deblocking reaction. |
| 29 | H | $CF_3CH_2$ | $CH_3$ | PNB, H, Na | |

| Compound | $R^6$ | $R^7$ | $R^3$ | $R^4$ | Remarks |
|---|---|---|---|---|---|
| 30 | H | $CH_3O_2CCH_2$ | $CH_3$ | PNB, H, Na | |
| 31 | H | $CH_3O_2CCH_2$ | $CH_3$ | phthalidyl | |
| 32 | H | $CH_3O_2CCH_2$ | $CH_3$ | $CH_2OCOC(CH_3)_2$ | |
| 33 | H | 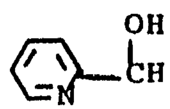 | $CH_3$ | PNB, H | Hydroxyl group protected as $PNBO_2C$ derivative. Deprotected at ester deblocking stage. |
| 34 | H | 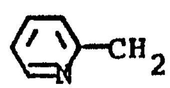 | $CH_3$ | PNB, H | |

| Compound | $R^6$ | $R^7$ | $R^3$ | $R^4$ | Remarks |
| --- | --- | --- | --- | --- | --- |
| 35 | H | $CH_3CH_2\overset{OH}{CH}$ | $CH_3$ | PNB, H, Na | Hydroxyl group protected as $PNBO_2C$ derivative. Deprotection occurs in ester deblocking reaction. |
| 36 | H | $CF_3\overset{OH}{CH}$ | $CH_3CH_2$ | PNB, H, Na | " |
| 37 | H | 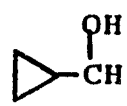 | $CH_3CH_2$ | PNB, H, Na | " |
| 38 | H | $CF_3$ | $CH_2C_6H_5$ | PNB, H, Na | |

| Compound | $R^6$ | $R^7$ | $R^3$ | $R^4$ | Remarks |
|---|---|---|---|---|---|
| 39 | H | $HOCH_2CH_2CH_2$ | $CH_3$ | PNB, H, Na | Hydroxyl group is protected as $PNBO_2C$ derivative which is cleaved in ester deblocking reaction. |
| 40 | H | $C_6H_5CH_2$ | $CH_3CH_2$ | PNB, H, K | $KHCO_3$ replaces $NaHCO_3$ in ester reduction reaction. |
| 41 | H | $(CH_3)_2CH$ | $CH_3CH_2$ | PNB, H, Na | |

| Compound | $\underline{R}^6$ | $\underline{R}^7$ | $\underline{R}^3$ | $\underline{R}^4$ | Remarks |
|---|---|---|---|---|---|
| 42 | H | $CH_3O$ | $CH_3CH_2$ | $CH_2CH=CH_2$, K | |
| 43 | H | $HOCH_2$ | $CH_3CH_2$ | $CH_2CH=CH_2$, K | The hydroxyl group is protected as its t-butyl-dimethylsilyl ether which is deprotected using $Bu_4NF$/HOAc either prior to or after sulfur oxidation and before ester deprotection. |
| 44 | H | $FCH_2\overset{HO}{CH}$ | $CH_3CH_2$ | $CH_2CH=CH_2$, K | " |
| 45 | H | $(CH_3)_2\overset{OH}{C}$ | $CH_3CH_2$ | $CH_2CH=CH_2$, K | " |
| 46 | H | $CH_3CH_2\overset{HO}{CH}$ | $CH_3$ | $CH_2CH=CH_2$, K | " |

EXAMPLE 11

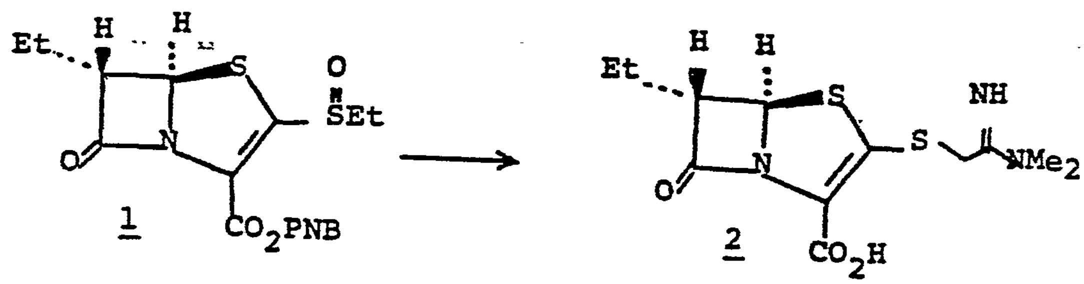

1

2

Preparation of ±-(5R,6S)-6-Ethyl-2-[N,N-dimethylcarbamimidoylmethylthio]-pen-2-em-3-carboxylate

To a stirred solution of 82.0 mg (0.2 mmol) of penem sulfoxide 1 in 1.5 ml of DMF at -20°C under an atmosphere of nitrogen is added a solution of 30.8 mg (0.2 mmol) of N,N-dimethyl-2-mercaptoacetamidium hydrochloride in 0.5 ml DMF and 25.8 mg (0.2 mmol) of diisopropylethylamine. The mixture is stirred at -20°C under $N_2$ for 5 minutes and the resulting transformed penem precipitated by the addition of $Et_2O$. After decantation of the supernatant, the separated product is washed analogously with $Et_2O$ and dried briefly *in vacuo*. The residue is then dissolved in 4 ml of THF, 2 ml $H_2O$, and 0.5 ml of 0.1M pH 7.1 phosphate buffer, and hydrogenated with 40 mg of $PtO_2$ catalyst at 50 psi at ambient temperature for 3.5 hours. The catalyst is removed by filtration through filter aid, washed well with EtOAc and $H_2O$. The filtrate is further diluted with EtOAc and $H_2O$ and the aqueous is separated, washed further with EtOAc, and concentrated *in vacuo*. The concentrate is purified by reverse phase chromatography to give the final product 2.

## EXAMPLE 12

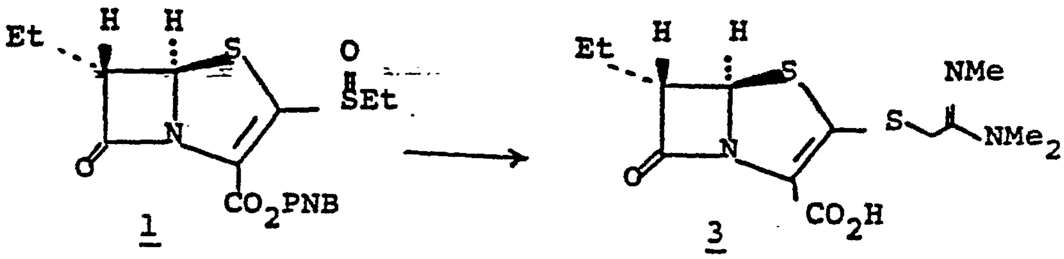

1 3

Preparation of (±)-(5R,6S)-6-Ethyl-2-[N,N,N'-trimethylcarbamimidoylmethylthio]-pen-2-em-3-carboxylate

To a stirred solution of 100 mg (0.24 mmols) of penem sulfoxide 1 in 1.8 ml of DMF at -20°C under an atmosphere of nitrogen is added a solution of 52.8 mg (0.24 mmols) of N,N,N'-trimethyl-2-mercapto-acetamidinium tetrafluoroborate in 0.6 ml DMF and 31.0 mg (0.24 mmols) of diisopropylethylamine. The mixture is stirred at -20°C under nitrogen for 5 minutes and diethylether is then added to effect the separation of the adduct. After washing and drying *in vacuo*, the adduct is dissolved in 4 ml THF, 2 ml $H_2O$, and 0.5 ml of 0.1M pH 7.1 phosphate buffer and is hydrogenated with 50 mg of $PtO_2$ catalyst at 50 psi on a Parr apparatus for 3.0 hours. The final product 3 is isolated by conventional techniques.

EXAMPLE 13

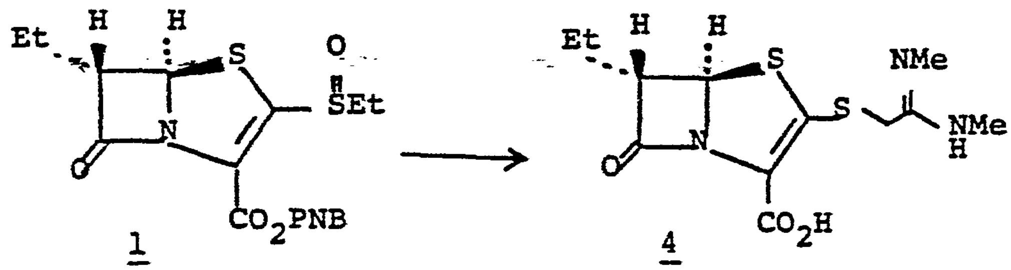

1 4

Preparation of (±)-(5R,6S)-6-Ethyl-2-[N,N'-dimethyl-carbamimidoylmethylthio]-pen-2-em-3-carboxylate

To a stirred solution of 123.0 mg (0.3 mmol) of penem sulfoxide 1 in 2 ml of DMF at -20°C under an atmosphere of nitrogen is added a solution of 61.8 mg (0.3 mmol) of N,N'-dimethyl-2-mercaptoacetamidinium tetrafluoroborate in 0.75 ml DMF and 38.8 mg (0.3 mmol) of diisopropylethylamine. The mixture is stirred at -20°C for 5 minutes and diethyl ether is then added to separate the transformed penem. The supernatant is removed by decantation and the separated material washed with $Et_2O$ (2X) and dried *in vacuo* for a short time. The material is then dissolved in 4 ml THF, 2 ml $H_2O$, and 0.5 ml of 0.1M pH 7.1 phosphate buffer. The solution is shaken with 25 mg of $PtO_2$ catalyst on a Parr apparatus under 50 psig of hydrogen for 4.0 hours. After this time the final product 4 is isolated by conventional procedures.

EXAMPLE 14

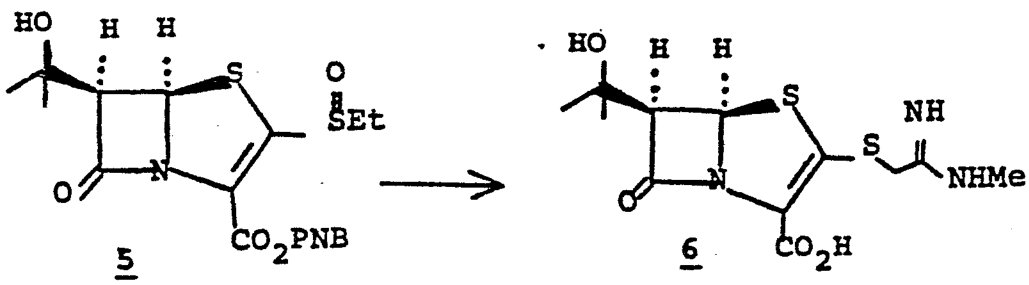

Preparation of (±)-(5R,6R)-6-[1'-hydroxyisopropyl]-2-[N-methylcarbamimidoylmethylthio]-pen-2-em-3-carboxylate

To a stirred solution of 88.0 mg (0.2 mmol) of penem sulfoxide 5 in 2.0 ml of DMF at -35°C under an atmosphere of nitrogen is added a solution of 28.0 mg (0.2 mmol) of N-methyl-2-mercaptoacetamidinium hydrochloride in 0.5 ml DMF and 25.8 mg (0.2 mmol) of diisopropylethylamine. The mixture is stirred at -35°C for 5 minutes and diethylether is then added. The supernatant is decanted away from the separated material which is then washed analogously with $Et_2O$. The material is dissolved in 4 ml THF, 2 ml $H_2O$, and 0.5 ml of pH 7.1 phosphate buffer and 30 mg of $PtO_2$ catalyst is added. The mixture is hydrogenated on a Parr apparatus at 50 psi and ambient temperature for 2.5 hours. After this time the product 6 is isolated by conventional procedures.

EXAMPLE 15

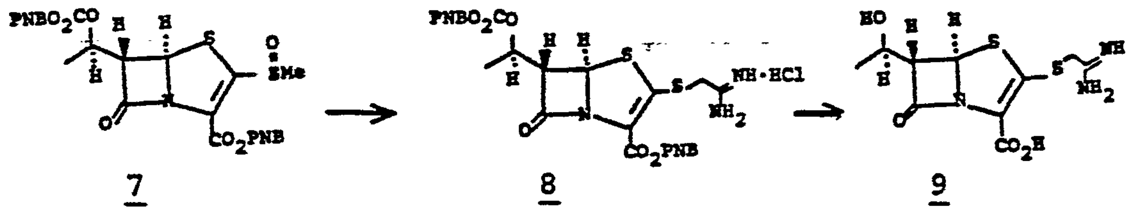

7 8 9

(5R,6S)-2-Carbamimidoylmethylthio-6-[(R)-1-hydroxypropyl]-pen-2-em-3-carboxylic acid (9)

Bis protected sulfoxide 7 (60.6 mg, 0.1 mmol) in anhydrous DMF (0.8ml) is stirred in a dry ice-$CCl_4$ bath (-23°) and under a $N_2$ atmosphere. A solution of carbmimidoylmethyl mercaptan hydrochloride (12.7 mg, 0.1 mmol) in DMSO (0.2 ml) and $iPr_2NEt$ (19.2 ml, 0.11 mmol) are added dropwise and simultaneously. The resulting mixture is stirred for 10 minutes at -20°, then added to $Et_2O$ (16 ml) and shaken. The $Et_2O$ phase is decanted from the oily residue of crude displacement product 8 which is washed with more $Et_2O$ (2 X 6 ml). The oily residue is taken up in THF (4 ml) and 0.1M pH 7 phosphate buffer (3 ml) and hydrogenated at atmospheric pressure with 10% PdlC (30 mg). After 1 hour, more catalyst (30 mg) is added and the hydrogenation is continued for an additional 1 hour. The mixture is filtered and the catalyst is washed with $H_2O$ (2 X 5 ml). The filtrate is washed with EtOAc (2 X 10 ml), concentrated under vacuum to ca. 2 ml, and streaked on two 0.25 mm X 20 X 20 cm. Analtech RPS-f plates which are developed with 10% $EtOH/H_2O$ in a cold room. The major UV visible zone is removed and eluted with 4:1 $MeCN-H_2O$ (4 X 10 ml). The eluant

is washed with hexanes, (2 X 20 ml) concentrated under vacuum, and lyophilized to provide the title compound <u>9</u>.

EXAMPLE 16

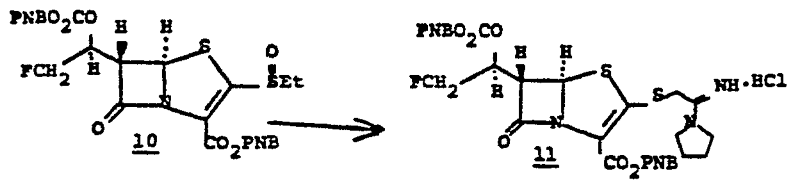

<u>10</u> <u>11</u>

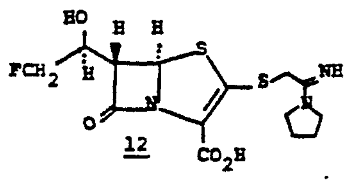

<u>12</u>

(5R,6S)-2-(N,N-tetramethylene)carbamimidoylmethylthio-6-[(S)-1-hydroxy-2-fluoroethyl]-pen-2-em-3-carboxylic acid (<u>12</u>)

A solution of sulfoxide <u>10</u> (56 mg, 0.09 mmol) in DMF (0.6 ml) is cooled in a dry ice -$CCl_4$ bath and stirred under a $N_2$ atmosphere. A solution of (N,N-tetramethylene)carbamimidoylmethyl mercaptan hydrochloride (16.3 mg, 0.09 mmol) in DMSO (0.15 ml) is added followed by $iPr_2NEt$ (15.7 ml, 0.09 mmol) and the resulting solution is stirred at ca. -20° for 15 minutes. The solution is added to $Et_2O$ (10 ml) and centrifuged to give an oily precipitate of crude <u>11</u>. The precipitate is washed with $Et_2O$ (2 X 5 ml), taken up in THF (3.6 ml) and 0.1M pH 7.1 potassium phosphate buffer (2.7 ml) and hydrogenated with 10% Pd/C (50 mg) at 45 psi for 2 hours. The mixture is filtered and the catalyst is washed with $H_2O$ (2 X 5 ml). The aqueous filtrate is washed with EtOAc (3 X 10 ml), concentrated under vacuum to

ca. 2 ml, and changed onto a Dowex 50 X 2 column (40 ml,K form). The column is eluted with $H_2O$ in a cold room. The product fractions are located by UV, combined, concentrated, and lyophilized to yield the title compound 12.

EXAMPLE 17

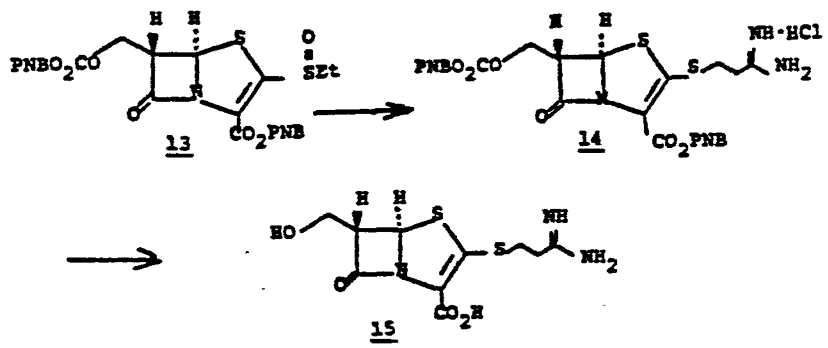

13 14 15

(5R,6S)-2-Carbamimidoylethylthio-6-hydroxymethyl-pen-2-em-3-carboxylic acid (15)

Sulfoxide 13 (27.2 mg, 0.046 mmol) is dissolved in anhydrous DMF (0.35 ml) under a $N_2$ atm. and the solution is cooled in a dry ice -$CCl_4$ bath. Carbamimidoylethylmercaptan hydrochloride (5 mg, 0.046 mmol) in DMSO (0.1 ml) and i$Pr_2$NEt (8.7 ml, 0.05 mmol) are added and the resulting solution is stirred for 15 minutes at -20°. The solution is added to $Et_2O$ (8 ml) and shaken. The $Et_2O$ phase is decanted from the oily residue which is washed with more $Et_2O$ (2 X 3 ml). The oily residue of crude 14 is taken up in THF (1.8 ml) and 0.1M pH 7 potassium phosphate buffer (1.4 ml) and hydrogenated at 45 psi with 10% Pd/C (50 mg) for 2 hours. The mixture is filtered and the catalyst is washed with $H_2O$ (2 X 3 ml). The aqueous filtrate is washed with EtOAc (2 X 5 ml), concentrated *in vacuo* to ca. 1 ml, and streaked on a 0.25 mm X 20 X 20 cm Analtech RPS-F plate which is developed with 10% $EtOH/H_2O$.

The desired UV visible band is removed and extracted with 4:1 MeCN-$H_2O$ (4 X 5 ml). The extracts are washed with hexanes (2 X 10 ml), concentrated in vacuo, and lyophilized to afford the title compound 15.

## EXAMPLE 18

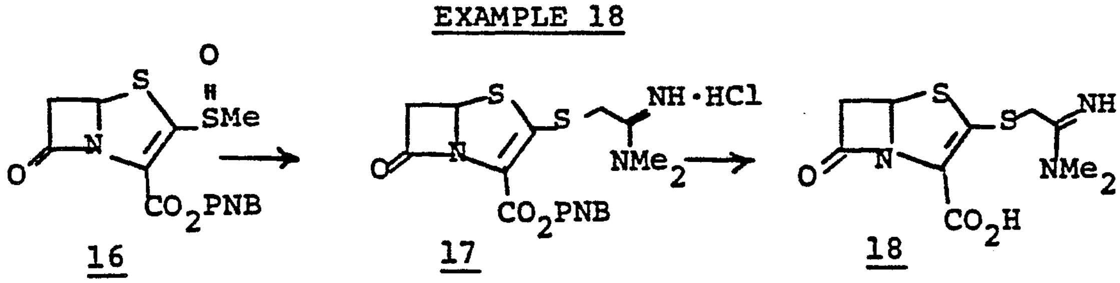

16 17 18

2-(N,N-Dimethyl)carbamimidoylmethylthio-pen-2-em-3-carboxylic acid (18)

A solution of sulfoxide 16 (1.47 g, 4 mmol) in anhydrous DMF (12 ml) is cooled in a dry ice $-CCl_4$ bath under a $N_2$ atm. and treated simultaneously and dropwise over 4 minutes with $iPr_2NEt$ (0.77 ml, 4.4 mmol) and a solution of (N,N-dimethylcarbamimidoylmethyl mercaptan hydrochloride (0.62 g, 4 mmol) in DMSO (8 ml). After stirring for 10 minutes at -20°, the mixture is added to a rapidly stirring mixture of supercel (16 g) in $Et_2O$ (400 ml). The mixture is stirred for a few minutes and filtered. The supercel pad is washed with more $Et_2O$ (400 ml) and sucked dry under a rubber dam.

The crude intermediate 17 is extracted from the supercel by successive washing with THF (100 ml)-$H_2O$(30 ml), THF (20 ml), and 0.1M pH 7.1 phosphate buffer (110 ml). The combined extracts are divided into two equal portions and each is hydrogenated with 10% Pd/C (0.7 g) for 2 hours at 45

psi. The mixture is filtered and the filtrate is washed with $Et_2O$ (2 X 250 ml). The aqueous portion is concentrated under vacuum and charged onto a Dowex 50W X 4 column (500 ml, Na form, 200-400 mesh) which is eluted with $H_2O$ in a cold room. The appropriate fractions, as determined by UV spectroscopy, are combined, concentrated under vacuum, and lyophilized to afford the title compound 18.

EXAMPLE 19

Following the procedures of the foregoing text and examples, the following "carbamimidoyl" embodiments of the present invention are obtained.

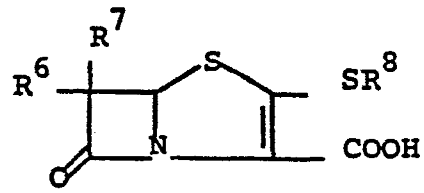

| Compound | $R^6$ | $R^7$ | $R^8$ |
|---|---|---|---|
| 19 | H | $CH_3CH_2$ | 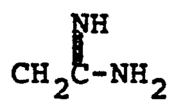 |
| 20 | H | $CH_3CH_2$ | 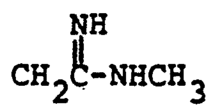 |
| 21 | H | $CH_3CH_2$ | 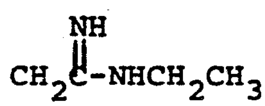 |
| 22 | H | $CH_3CH_2$ | 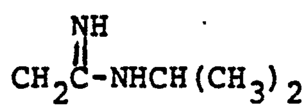 |
| 23 | H | $CH_3CH_2$ | 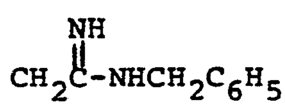 |
| 24 | H | $CH_3CH_2$ | 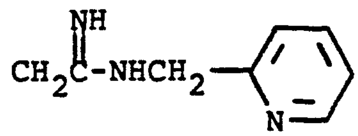 |
| 25 | H | $CH_3CH_2$ | 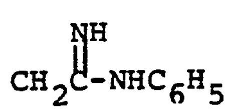 |

| Compound | $R^6$ | $R^7$ | $R^8$ |
|---|---|---|---|
| 26 | H | $CH_3CH_2$ | $CH(CH_3)\overset{NH}{\overset{\|}{C}}-NH_2$ |
| 27 | H | $CH_3CH_2$ | $CH(CH_3)\overset{NH}{\overset{\|}{C}}-NHCH_3$ |
| 28 | H | $CH_3CH_2$ | $CH(CH_3)\overset{NH}{\overset{\|}{C}}-N(CH_3)_2$ |
| 29 | H | $CH_3CH_2$ | $CH(CH_2CH_3)\overset{NH}{\overset{\|}{C}}-NH_2$ |
| 30 | H | $CH_3CH_2$ | $CH_2CH_2\overset{NH}{\overset{\|}{C}}-NH_2$ |
| 31 | H | $CH_3CH_2$ | $CH_2CH_2\overset{NH}{\overset{\|}{C}}-N(CH_3)_2$ |
| 32 | H | $CH_3CH_2$ | $CH_2CH_2CH_2\overset{NH}{\overset{\|}{C}}-NH_2$ |
| 33 | H | $CH_3CH_2$ | 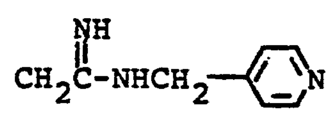 |
| 34 | H | $CH_3CH_2$ | $CH_2\overset{NH}{\overset{\|}{C}}-N(CH_3)CH_2CH_3$ |
| 35 | H | $CH_3CH_2$ | 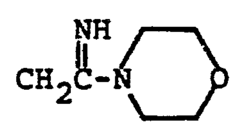 |

| Compound | $R^6$ | $R^7$ | $R^8$ |
|---|---|---|---|
| 36 | H | $CH_3CH_2$ | 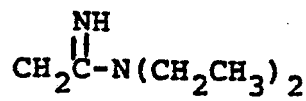 |
| 37 | H | $CH_3CH_2$ | 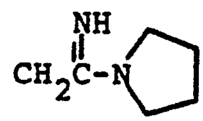 |
| 38 | H | $CH_3CH_2$ | 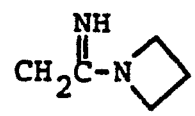 |
| 39 | H | $CH_3CH_2$ | 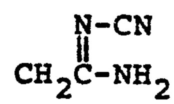 |
| 40 | H | $CH_3CH_2$ | 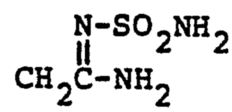 |
| 41 | H | $CH_3CH_2$ | 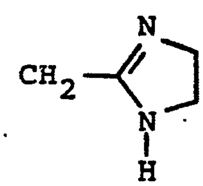 |
| 42 | H | $CH_3CH_2$ | 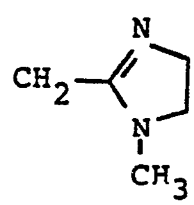 |

| Compound | $R^6$ | $R^7$ | $R^8$ |
|---|---|---|---|
| 43 | H | $CH_3CH_2$ | 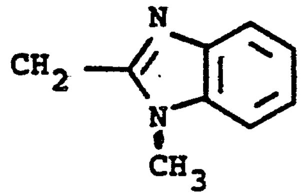 |
| 44 | H | $CH_3CH_2$ | 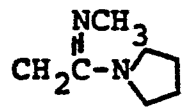 |
| 45 | H | $CH_3CH_2$ | 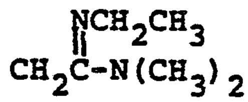 |
| 46 | H | $CH_3CH_2$ | 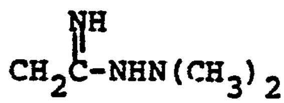 |
| 47 | H | $CH_3CH_2$ | 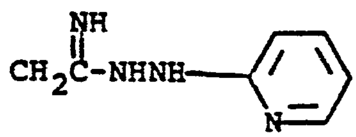 |
| 48 | H | $CH_3CH_2$ | 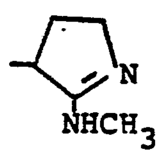 |
| 49 | H | $CH_3CH_2$ | 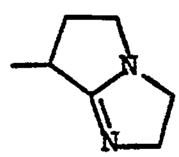 |

| Compound | $R^6$ | $R^7$ | $R^8$ |
|---|---|---|---|
| 50 | H | $CH_3CH_2$ | 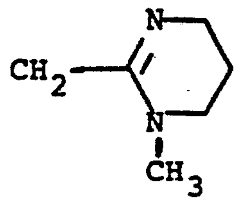 |
| 51 | H | $CH_3CH_2$ | 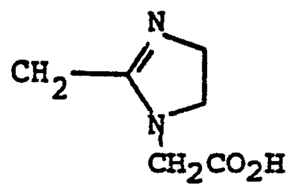 |
| 52 | H | $HOCH_2$ | $CH_2\overset{NH}{\overset{\|}{C}}-NH_2$ |
| 53 | H | $HOCH_2$ | $CH(CH_3)\overset{NH}{\overset{\|}{C}}-NH_2$ |
| 54 | H | $HOCH_2$ | $CH_2\overset{NH}{\overset{\|}{C}}-NHCH_3$ |
| 55 | H | $HOCH_2$ | $CH_2\overset{NH}{\overset{\|}{C}}-N(CH_3)_2$ |
| 56 | H | $HOCH_2$ | $CH_2\overset{NCH_3}{\overset{\|}{C}}-NHCH_3$ |
| 57 | H | $HOCH_2$ | $CH_2\overset{NCH_3}{\overset{\|}{C}}-N(CH_3)_2$ |
| 58 | H | $HOCH_2$ | $CH_2\overset{NH}{\overset{\|}{C}}-NHCH_2CH_3$ |
| 59 | H | $HOCH_2$ | $CH_2CH_2\overset{NH}{\overset{\|}{C}}-N(CH_3)_2$ |

| Compound | $R^6$ | $R^7$ | $R^8$ |
|---|---|---|---|
| 60 | H | $HOCH_2$ | 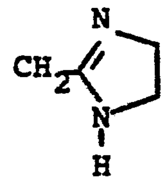 |
| 61 | H | $HOCH_2$ | 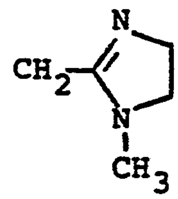 |
| 62 | H | $HOCH_2$ | $CH_2\overset{NH}{\overset{\|}{C}}-N(CH_3)CH_2CH_3$ |
| 63 | H | $HOCH_2$ | 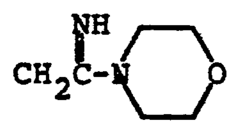 |
| 64 | H | $HOCH_2$ | 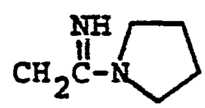 |
| 65 | H | $HOCH_2$ | 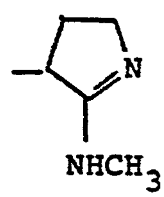 |
| 66 | H | $FCH_2CH(OH)$ | $CH_2\overset{NH}{\overset{\|}{C}}-NH_2$ |

| Compound | $R^6$ | $R^7$ | $R^8$ |
|---|---|---|---|
| 67 | H | $FCH_2CH(OH)$ | 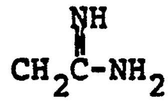 |
| 68 | H | $FCH_2CH(OH)$ | 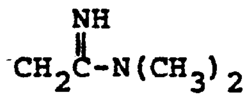 |
| 69 | H | $FCH_2CH(OH)$ | 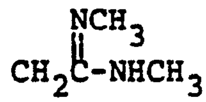 |
| 70 | H | $FCH_2CH(OH)$ | 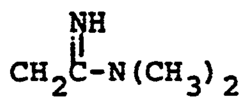 |
| 71 | H | $FCH_2CH(OH)$ | 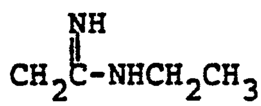 |
| 72 | H | $FCH_2CH(OH)$ | 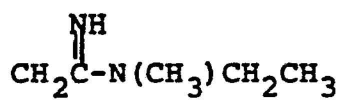 |
| 73 | H | $FCH_2CH(OH)$ | 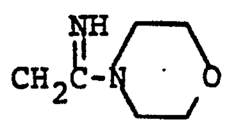 |
| 74 | H | $FCH_2CH(OH)$ | 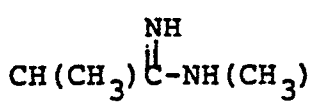 |
| 75 | H | $FCH_2CH(OH)$ | 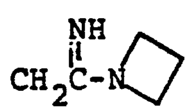 |
| 76 | H | $FCH_2CH(OH)$ | 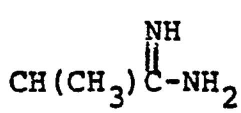 |

| Compound | $R^6$ | $R^7$ | $R^8$ |
|---|---|---|---|
| 77 | H | $FCH_2CH(OH)$ | 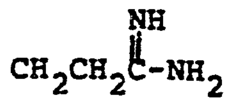 |
| 78 | H | $FCH_2CH(OH)$ | 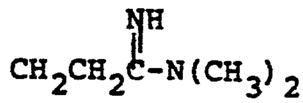 |
| 79 | H | $FCH_2CH(OH)$ | 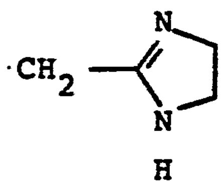 |
| 80 | H | $FCH_2CH(OH)$ | 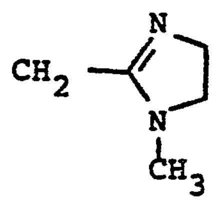 |
| 81 | H | $FCH_2CH(OH)$ | 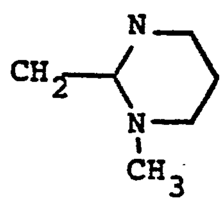 |
| 82 | H | $FCH_2CH(OH)$ | 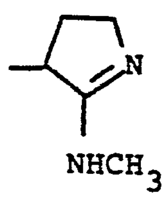 |

| Compound | $R^6$ | $R^7$ | $R^8$ |
|---|---|---|---|
| 83 | H | $CH_3CH_2CH(OH)$ | 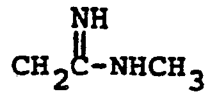 |
| 84 | H | $CH_3CH_2CH(OH)$ | 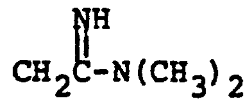 |
| 85 | H | $CH_3CH_2CH(OH)$ | 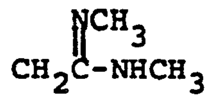 |
| 86 | H | $CH_3CH_2CH(OH)$ | 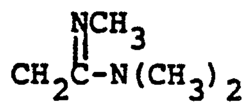 |
| 87 | H | $CH_3CH_2CH(OH)$ | 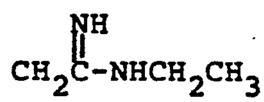 |
| 88 | H | $CH_3CH_2CH(OH)$ | 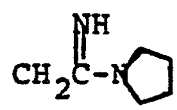 |
| 89 | H | $CH_3CH_2CH(OH)$ | 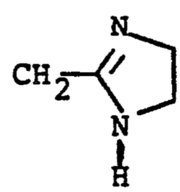 |
| 90 | H | $(CH_3)_2C(OH)$ | 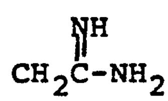 |
| 91 | H | $(CH_3)_2C(OH)$ | 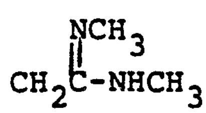 |

| Compound | $R^6$ | $R^7$ | $R^8$ |
|---|---|---|---|
| 92 | H | $(CH_3)_2C(OH)$ | NH<br>‖<br>$CH_2C-N(CH_3)_2$ |
| 93 | H | $(CH_3)_2C(OH)$ | $NCH_3$<br>‖<br>$CH_2C-N(CH_3)_2$ |
| 94 | H | 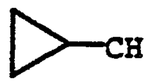CH(OH) | NH<br>‖<br>$CH_2C-NH_2$ |
| 95 | H | 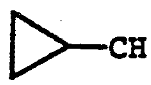CH(OH) | NH<br>‖<br>$CH_2C-N(CH_3)_2$ |
| 96 | H | 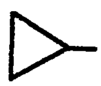 CH(OH) | NH<br>‖<br>$CH_2C$-N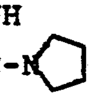 |
| 97 | H | 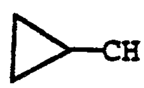CH(OH) | $NCH_3$<br>‖<br>$CH_2C-N(CH_3)_2$ |
| 98 | H | 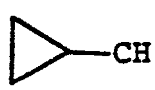CH(OH) | $CH_2$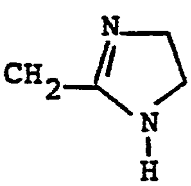 |
| 99 | H | $CH_3O$ | NH<br>‖<br>$CH_2C-N(CH_3)_2$ |

| Compound | $R^6$ | $R^7$ | $R^8$ |
|---|---|---|---|
| 100 | $CH_3CH(OH)$ | $CH_3O$ | 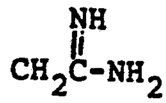 |
| 101 | $CH_3CH(OH)$ | $CH_3O$ | 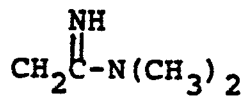 |
| 102 | $CH_3CH(OH)$ | $CH_3O$ | 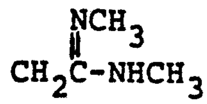 |
| 103 | $CH_3CH(OH)$ | $CH_3O$ | 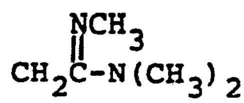 |
| 104 | $CH_3CH(OH)$ | $CH_3O$ | 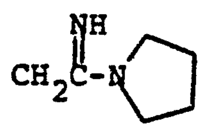 |
| 105 | H | $(CH_3)_2CH$ | 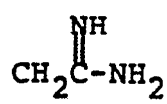 |
| 106 | H | $(CH_3)_2CH$ | 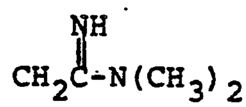 |
| 107 | H | $HO_2CCH_2$ | 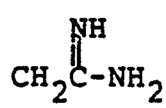 |
| 108 | H | 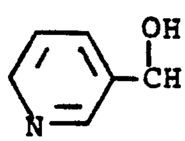 | 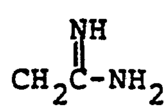 |

| Compound | $R^6$ | $R^7$ | $R^8$ |
|---|---|---|---|
| 109 | $CF_3$ | 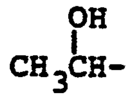 | 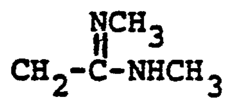 |
| 110 | $CF_3$ | 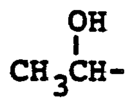 | 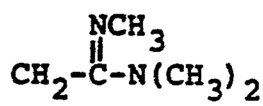 |
| 111 | $CH_3O$ | 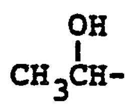 | 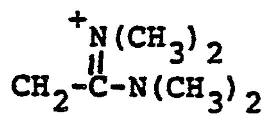 |
| 112 | $CH_3O$ | 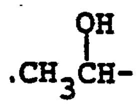 | 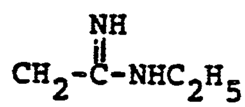 |
| 113 | $CH_3O$ | 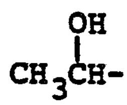 | 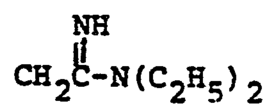 |
| 114 | $CH_3O$ | 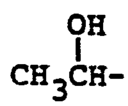 | 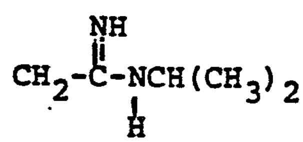 |
| 115 | $CH_3O$ | 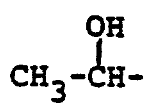 | 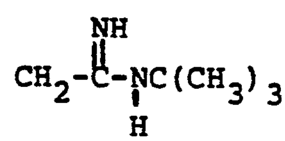 |
| 116 | $CH_3O$ | 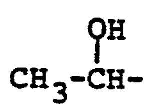 | 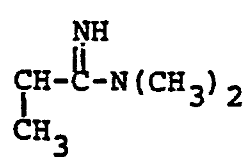 |
| 117 | $CF_3$ | 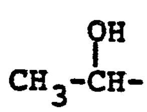 | 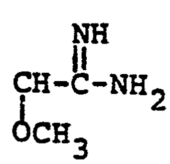 |

| Compound | $R^6$ | $R^7$ | $R^8$ |
|---|---|---|---|
| 118 | $CH_3O$ | 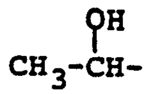 | 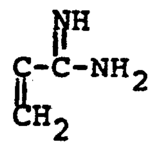 |
| 119 | $CH_3O$ | 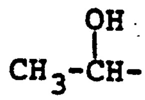 | 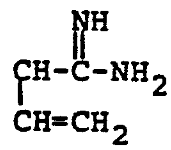 |
| 120 | $CH_3O$ | 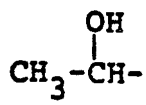 | 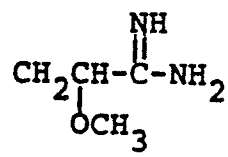 |
| 121 | $CH_3$ | 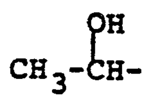 | 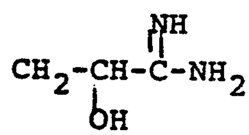 |
| 122 | $CH_3$ | 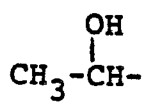 | 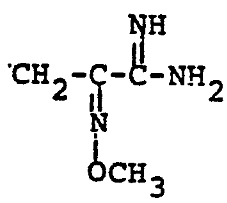 |
| 123 | $CH_3$ | 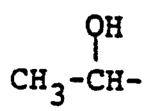 | 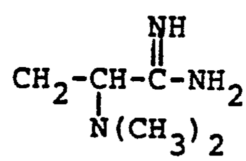 |
| 124 | $CH_3$ | 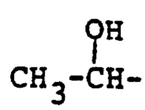 | 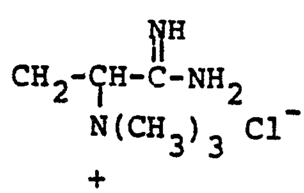 |

| Compound | $R^6$ | $R^7$ | $R^8$ |
|---|---|---|---|
| 125 | $CH_3O$ | 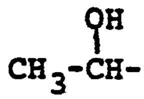 | 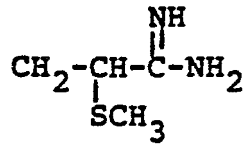 |
| 126 | $HOCH_2$ | 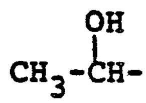 | 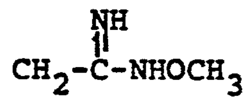 |
| 127 | $CH_3O$ | 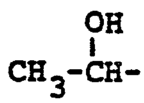 | 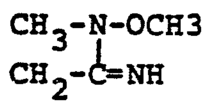 |
| 128 | $CF_3$ | 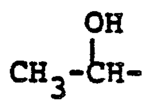 | 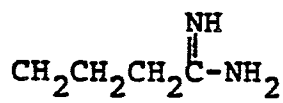 |
| 129 | $CF_3$ | 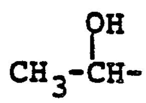 | 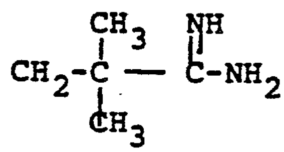 |
| 130 | $CH_3O$ | 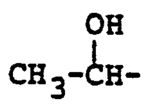 | 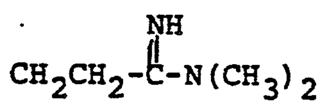 |
| 131 | $CF_3$ | 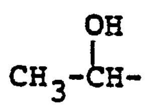 | 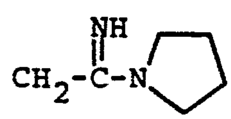 |
| 132 | $CH_3O$ | 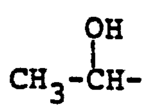 | 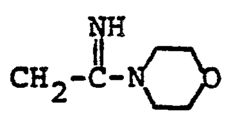 |
| 133 | $HOCH_2$ | 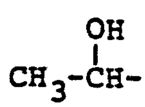 | 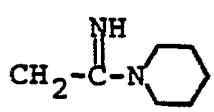 |

| Compound | $R^6$ | $R^7$ | $R^8$ |
|---|---|---|---|
| 134 | $CF_3$ | 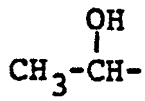 | 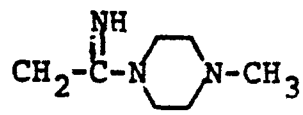 |
| 135 | $CF_3$ | 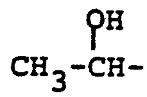 | 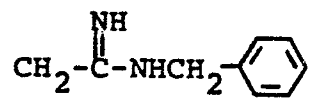 |
| 136 | $CF_3$ | 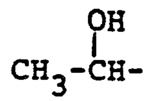 | 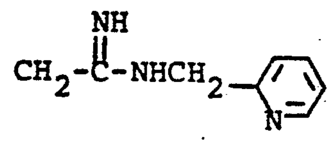 |
| 137 | $CF_3$ | 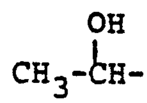 | 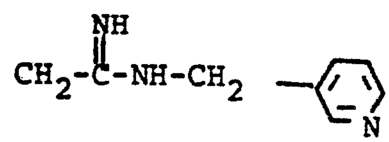 |
| 138 | $CF_3$ | 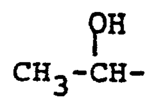 | 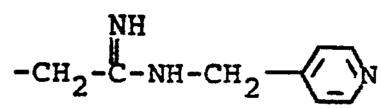 |
| 139 | $CH_3O$ | 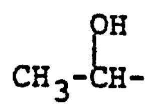 | 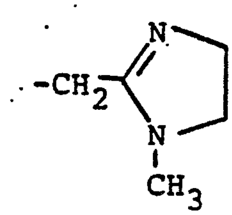 |
| 140 | $CH_3O$ | 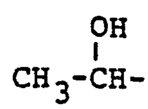 | 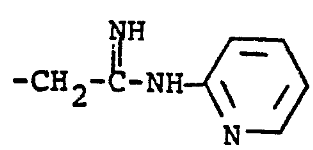 |
| 141 | $FCH_2$ | 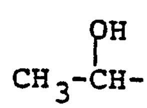 | 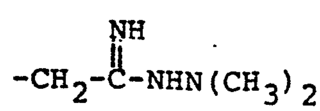 |

| Compound | $R^6$ | $R^7$ | $R^8$ |
|---|---|---|---|
| 142 | $FCH_2$ | 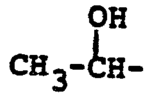 | 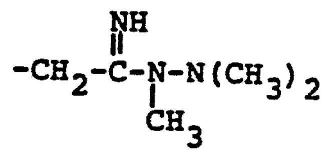 |
| 143 | $CH_3O$ | 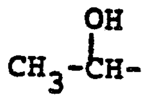 | 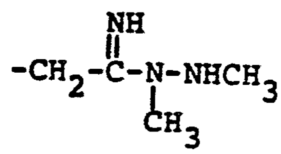 |
| 144 | $CF_3$ | 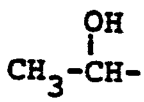 | 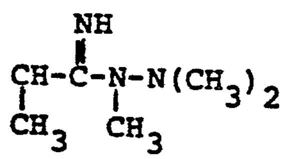 |
| 145 | $CF_3$ | 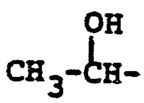 | 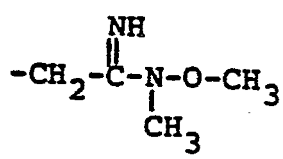 |
| 146 | F | 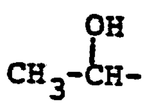 | 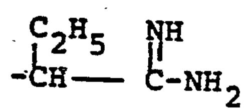 |
| 147 | F | 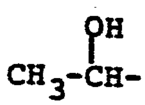 | 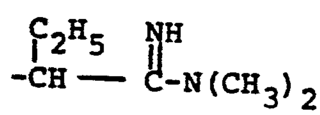 |
| 148 | F | 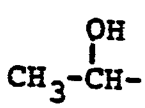 | 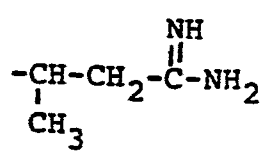 |
| 149 | F | 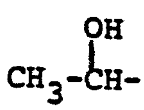 | 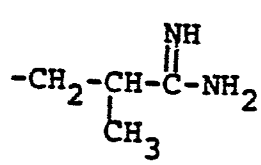 |

EXAMPLE 20

Preparation of Pharmaceutical Compositions

One such unit dosage form is prepared by mixing 120 mg of the compound of Example 16 (Compound A) with 20 mg of lctose and 5 mg of magnesium stearate and placing the 145 mg mixture into a No. 3 gelatin capsule. Similarly, by employing more of the active ingredient and less lactose, other odsage forms can be put up in No. 3 gelatin capsules, and, should it be necessary to make more than 145 mg of ingredients together, larger capsules such as compressed tablets and pills can be prepared. The following examples are illustrative of the preparation of pharmaceutical formulations:

| TABLET | PER TABLET |
|---|---|
| Compound A | 125 mg. |
| Cornstarch, U.S.P. | 6 mg. |
| Dicalcium Phosphate | 192 mg. |
| Lactose, U.S.P. | 190 mg. |
| Magnesium Stearate | Balance/800 mg. |

The active ingredient is blended with the dicalcium phosphate, lactose and about half of the cornstarch. The mixture is then granulated with 15% cornstarch paste (16 mg) and rough-screened. It is dried at 45°C and screened again through No. 16 screens. The balance of the cornstarch and magnesium stearate is added and the mixture is compressed into tablets, approximately 0.5 inch in diameter each weighing 800 mg.

| PARENTERAL SOLUTION | PER TABLET |
|---|---|
| Ampoule: | |
| Compound A | 500 mg. |
| Diluent: Sterile Water for Injection | 2 cc. |

OPHTHALMIC SOLUTION

| | | |
|---|---|---|
| Compound A | | 100 mg. |
| Hydropropylmethyl Cellulose | | 5 mg. |
| Sterile Water | to | 1 ml. |

OTIC SOLUTION

| | | |
|---|---|---|
| Compound A | | 100 mg. |
| Benzalkonium chloride | | 0.1 mg. |
| Sterile Water | to | 1 ml. |

TOPICAL OINTMENT

| | |
|---|---|
| Compound A | 100 mg. |
| Polyethylene Glycol 4000 U.S.P. | 400 mg. |
| Polyethylene Glycol 400 U.S.P. | 1.0 gram |

## EXAMPLE 21

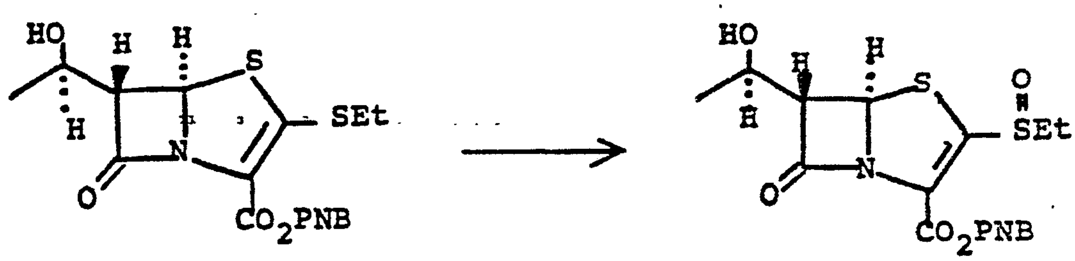

1 2

To a stirred solution of 300 mg (0.73 mmoles) of penem 1 in 5 ml of THF at -8°C under an atmosphere of nitrogen was added dropwise a solution of 178.3 mg (0.88 mmoles) of 85% m-chloroperbenzoic acid in 5 ml $CH_2Cl_2$ over a period of 15 min. After the addition, the mixture was stirred further for 15 min. The mixture was partitioned between EtOAc/ice-$H_2O$/5% aq. sodium thiosulfate solution. The organic phase was separated and washed further with cold, dilute sodium bicarbonate solution, and then a saturated solution of sodium chloride. The organic phase was dried over $Na_2SO_4$, filtered, and evaporated. Purification by plate layer chromatography [1 development $CH_2Cl_2$-EtOAc (1:1)] afforded 51.8 mg (17%) of unreacted penem 1 and 115.6 mg (37%) of sulfoxide 2 as a mixture of diastereomers: IR($CH_2Cl_2$) 3400, 1795, 1701, 1524, 1320 and 1056 $cm^{-1}$; UV (dioxane) 348 and 261 nm; NMR ($CDCl_3$) $\delta$ 1.39 (d, J=6.5Hz, $C\underline{H}_3CH$), 1.43 (t, J=7.5Hz, $C\underline{H}_3CH_2$), 3.12 (m, $CH_3C\underline{H}_2$), 3.95 (dd, J=2 and 6.5Hz, H6a), 3.98 (dd, J=2 and 6Hz, H6b), 4.30 (m, $CH_3C\underline{H}$), 5.27 and 5.49 (two d's, J=14Hz, $CH_2Ar$), 5.79 and 5.91 (two d's, J=2Hz, H5a and H5b), 7.64, 7.68 and 8.30 (three d's, J=8.5Hz, ArH). [Et = ethyl.]

## EXAMPLE 22

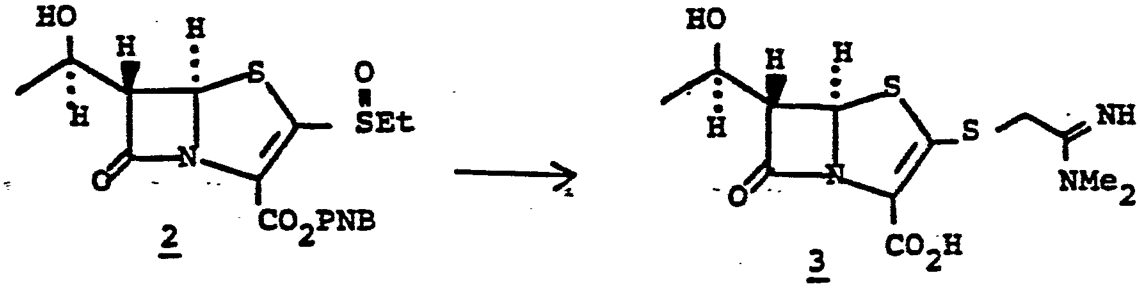

2 3

To a stirred solution of penem sulfoxide 2 (8.5 mg, .02 mmoles) in 1 ml THF and 0.25 ml of 0.1M pH 7.1 phosphate buffer at 0°C was added in rapid succession a soluton of 3.4 mg (0.022 mmoles) of N,N-dimethyl-2-mercaptoacetamidinium hydrochloride and 3.0 µl (0.022 mmoles) of diisopropylethylamine in 0.5 ml 0.1M pH 7.1 phosphate buffer and then 18 mg of 10% Pd/C. The mixture was hydrogenated at room temperature and 45 psi for 3.25 hours. The catalyst was removed by filtration and washed well with $H_2O$ and EtOAc. The filtrate was cooled in an ice-$H_2O$ bath and the aqueous phase was separated and washed with $Et_2O$. The aqueous phase was concentrated and purified by reverse phase plate layer chromatography [1 development 15% EtOH in $H_2O$] to give 1.65 mg (25.8%) of 3: $\lambda_{max}^{H_2O}$ 319 nm; NMR ($D_2O$) 1.29, 3H(d, J=6.5Hz); 3.16 3H(s); 3.31, 3H(s); 3.99, 1H(dd, J=1.5, 5.5Hz); 4.01, 1H(d, J=16.0Hz); 4.12, 1H(d, J=16Hz): 4.25, 1H(m); 5.69, 1H(d, J=1.5Hz). [Me = methyl.]

EXAMPLE 23

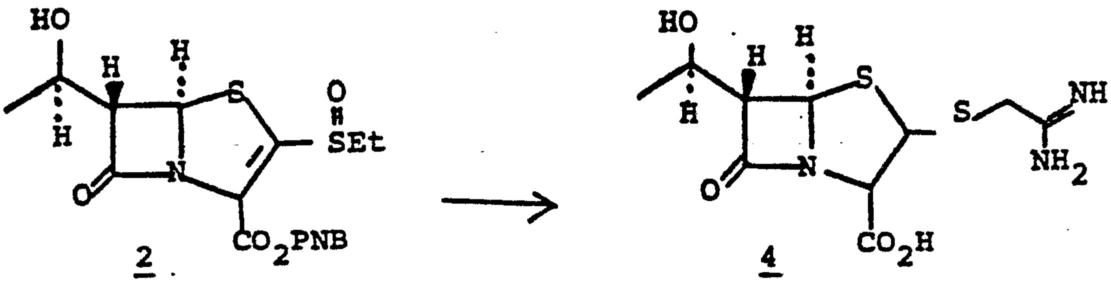

2 4

To a stirred solution of 7.6 mg (0.018 mmoles) of penem sulfoxide 2 in 1.0 ml THF and 0.25 ml of 0.1M pH 7.1 phosphate buffer at 0°C under a nitrogen atmosphere was added in rapid succession a solution of 2.5 mg (0.02 mmoles) of 2-mercapto-acetamidinium hydrochloride and 3.4 μl (0.02 mmoles) of diisopropylethylamine in 0.5 ml of 0.1M pH 7.1 phosphate buffer and 10 mg of 10% Pd/C. The mixture was hydrogenated at room temperature and 50 psi for 4.0 hours. The catalyst was removed by filtration and washed with EtOAc and $H_2O$. The aqueous phase was separated, washed with $Et_2O$, concentrated in vacuo, and purified by reverse phase plate layer chromatography [1 development 5% EtOH in $H_2O$] to give the product 4: $\lambda_{max}^{H_2O}$ 321.5 nm;

NMR ($D_2O$)$\delta$ 1.29, 3H(d, J=6.5Hz); 3.98, 1H(dd, J=1.5, 5.5Hz), 4.26, 1H(m), 5.72, 1H(d, J=1.5Hz).

EXAMPLE 24

Following the procedures of the foregoing text and examples, the following "carbamimidoyl" embodiments of the present invention are obtained.

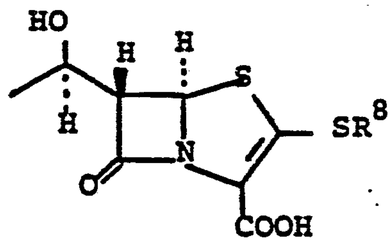

| Compound | R8 |
|---|---|
| 5 | 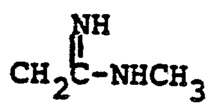 |
| 6 | 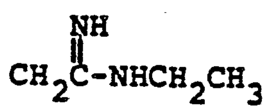 |
| 7 | 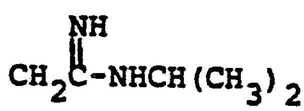 |

| Compound | $R^8$ |
|---|---|
| 8 | 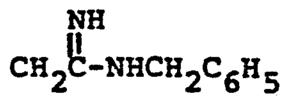 |
| 9 | 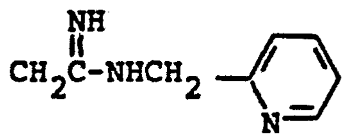 |
| 10 | 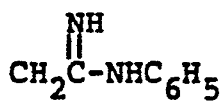 |
| 11 | 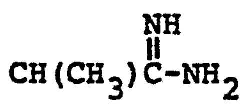 |
| 12 | 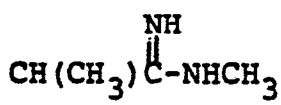 |
| 13 | 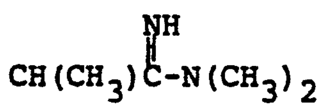 |
| 14 | 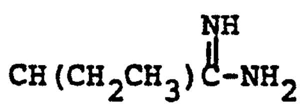 |
| 15 | 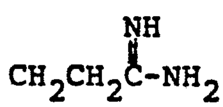 |
| 16 | 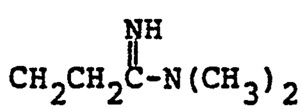 |
| 17 | 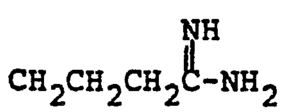 |

| Compound | $R^8$ |
|---|---|
| 18 | 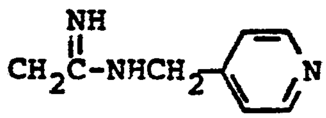 |
| 19 | 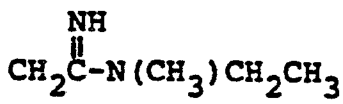 |
| 20 | 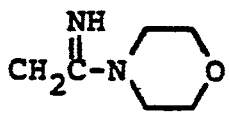 |
| 21 | 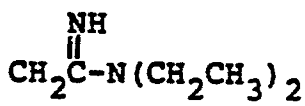 |
| 22 | 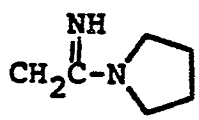 |
| 23 | 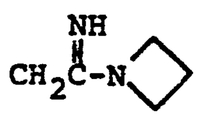 |
| 24 | 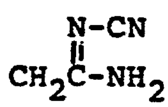 |
| 25 | 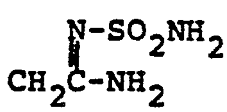 |

| Compound | $R^8$ |
|---|---|
| 26 | 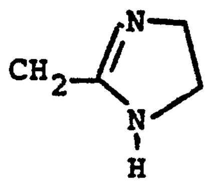 |
| 27 | 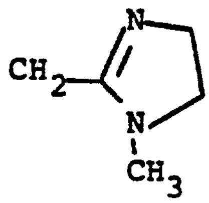 |
| 28 | 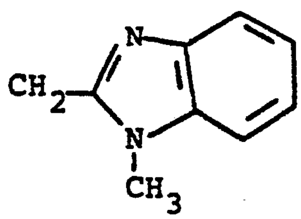 |
| 29 | 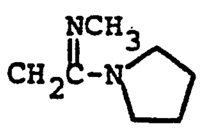 |
| 30 | 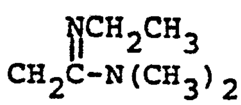 |
| 31 | 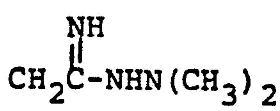 |
| 32 | 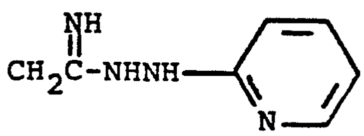 |

| Compound | $R^8$ |
|---|---|
| 33 | 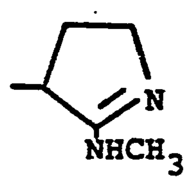 |
| 34 | 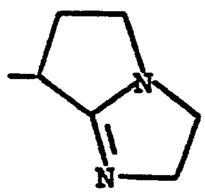 |
| 35 | 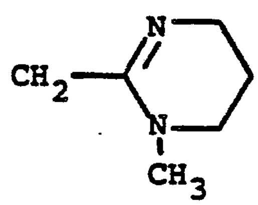 |
| 36 | 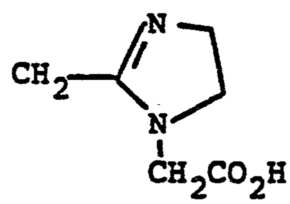 |
| 37 | $CH_2C(=NCH_3)-NHCH_3$ |
| 38 | $CH_2C(=NCH_3)-N(CH_3)_2$ |
| 39 | $CH_2CH_2C(=NH)-NHCH_3$ |
| 40 | $CH_2CH_2C(=NH)-NHCH_3$ |

| Compound | $R^8$ |
|---|---|
| 41 | 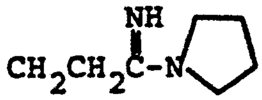 |
| 42 | 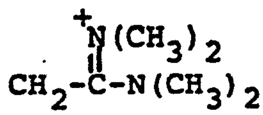 |
| 43 | 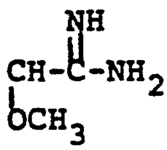 |
| 44 | 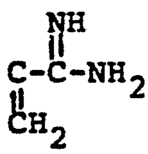 |
| 45 | 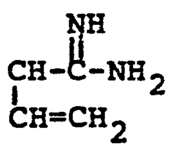 |
| 46 | 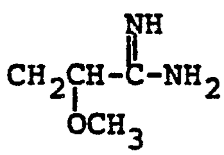 |
| 47 | 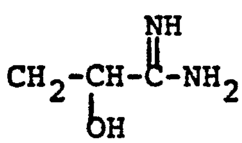 |

| Compound | $R^8$ |
|---|---|
| 48 | 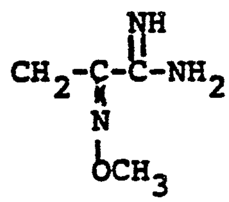 |
| 49 | 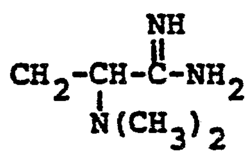 |
| 50 | 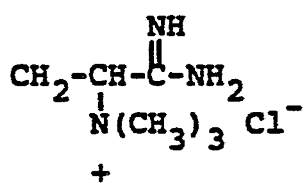 |
| 51 | 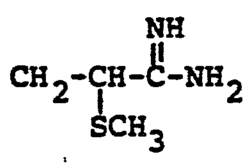 |
| 52 | 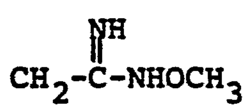 |
| 53 | 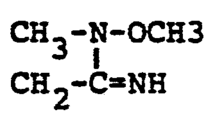 |
| 54 | 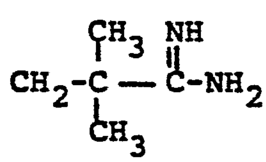 |

| Compound | $R^8$ |
|---|---|
| 55 | 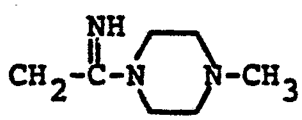 |
| 56 | 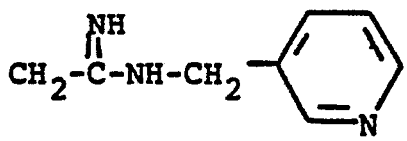 |
| 57 | 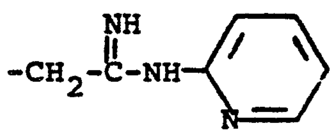 |
| 58 | 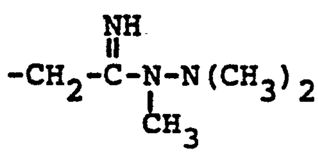 |
| 59 | 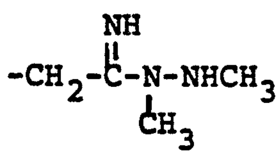 |
| 60 | 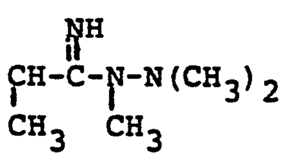 |
| 61 | 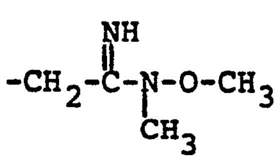 |
| 62 | 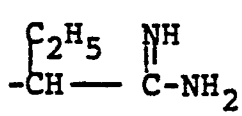 |

| Compound | $R^8$ |
|---|---|
| 63 | 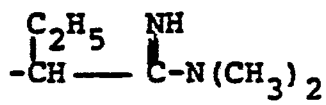 |
| 64 | 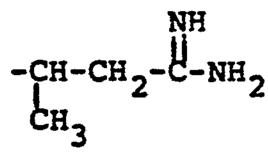 |
| 65 | 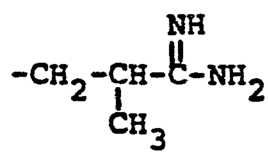 |

EXAMPLE 25

Preparation of Pharmaceutical Compositions

One such unit dosage form is prepared by mixing 120 mg of the compound of Example 22 (Compound 3) with 20 mg of lactose and 5 mg of magnesium stearate and placing the 145 mg mixture into a No. 3 gelatin capsule. Similarly, by employing more of the active ingredient and less lactose, other dosage forms can be put up in No. 3 gelatin capsules, and, should it be necessary to make more than 145 mg of ingredients together, larger capsules such as compressed tablets and pills can be prepared. The following examples are illustrative of the preparation of pharmaceutical formulations:

| TABLET | PER TABLET |
|---|---|
| Compound 3 | 125 mg. |
| Cornstarch, U.S.P. | 6 mg. |
| Dicalcium Phosphate | 192 mg. |
| Lactose, U.S.P. | 190 mg. |
| Magnesium Stearate | Balance/800 mg. |

The active ingredient is blended with the dicalcium phosphate, lactose and about half of the cornstarch. The mixture is then granulated with 15% cornstarch paste (16 mg) and rough-screened. It is dried at 45°C and screened again through No. 16 screens. The balance of the cornstarch and magnesium stearate is added and the mixture is compressed into tablets, approximately 0.5 inch in diameter each weighing 800 mg.

| PARENTERAL SOLUTION | | PER TABLET |
|---|---|---|
| Ampoule: | | |
| Compound 3 | | 500 mg. |
| Diluent: Sterile Water for Injection | | 2 cc. |

| OPHTHALMIC SOLUTION | | |
|---|---|---|
| Compound 3 | | 100 mg. |
| Hydropropylmethyl Cellulose | | 5 mg. |
| Sterile Water | to | 1 ml. |

| OTIC SOLUTION | | |
|---|---|---|
| Compound 3 | | 100 mg. |
| Benzalkonium chloride | | 0.1 mg. |
| Sterile Water | to | 1 ml. |

| TOPICAL OINTMENT | |
|---|---|
| Compound 3 | 100 mg. |
| Polyethylene Glycol 4000 U.S.P. | 400 mg. |
| Polyethylene Glycol 400 U.S.P. | 1.0 gram |

WHAT IS CLAIMED IS:

1. A compound having the structural formula:

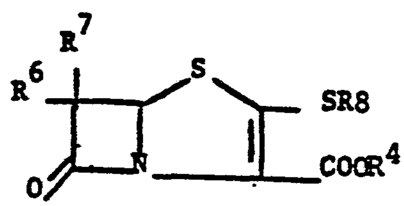

wherein $R^4$ is H, a carboxylate salt cation, removable protecting group, or a pharmaceutically acceptable salt, ester or amide moiety; wherein: $R^6$ and $R^7$, are independently selected from the group consisting of substituted and unsubstituted: hydrogen, alkyl, alkenyl, alkoxyl, and alkynyl having 1-10 carbon atoms; halo; hydroxyl; carboxyl; cyano; cycloalkyl, cycloalkylalkyl, and alkylcycloalkyl having 3-6 carbon atoms in the cycloalkyl ring and 1-6 carbon atoms in the alkyl moiety; aryl, aralkyl, aralkenyl, and aralkynyl wherein the aryl moiety is phenyl and aliphatic portion has 1-6 carbon atoms; heteroaryl, heteroaralkyl, heterocyclyl, and heterocyclylalkyl, wherein the alkyl moiety has 1-6 carbon atoms and the heteroatoms are selected from ON, N and S; $R^6$ and $R^7$ may be joined to form a cyclicalkyl having, together with the carbon atom to which they are attached, 3-6 carbon atoms; wherein the substituent or substituents on $R^6$, and $R^7$ are independently selected from chloro, fluoro, hydroxy, bromo, alkoxyl having 1-6 carbon atoms, mercapto, carboxyl, cyano, azido, amino, mono- and dialkylamino (each alkyl having 1-6 carbon atoms), ureido,

alkylthio having 1-6 carbon atoms; and $R^8$ is a carbamimidoyl selected from the group consisting of:

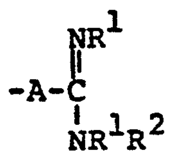 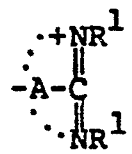 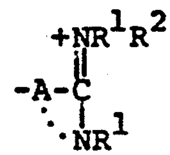

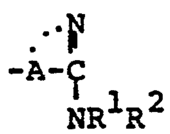 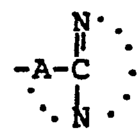 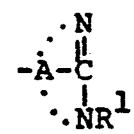

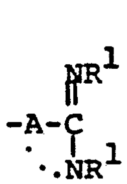 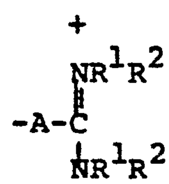 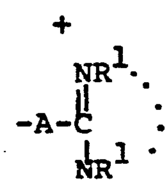

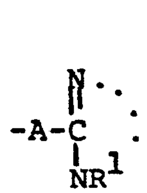 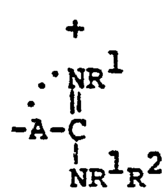 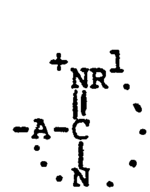

wherein: A is a single, direct bond, or A, the cyclic or acyclic connector, is selected from the group consisting of alkyl, alkenyl, and alkynyl having 1-10 carbon atoms which may be interrupted by a hetero atoms selected from O, S or N, or by a ring such as phenyl, cycloalkyl, cycloalkenyl, heterocyclyl or heteroaryl wherein such cyclic interruptions comprise 3-6 ring atoms selected from C, O, S and N; cycloalkyl, cycloalkenyl having 3-6 carbon atoms; heterocyclyl; heteroaryl; and phenyl; $R^1$ and $R^2$ are independently selected from:

hydrogen, $N(R^a)_2$, $OR^a$, ($R^a$ is hydrogen or $C_{1-6}$ alkyl), CN, $SO_2NH_2$ and the previously defined, but monovalent, values for the group A; and wherein the dotted lines indicate provision for cyclic structures formed by the joinder of the indicated nitrogen atom and the connector group A and by the joinder of the indicated nitrogen atoms.

2. A compound having the structural formula:

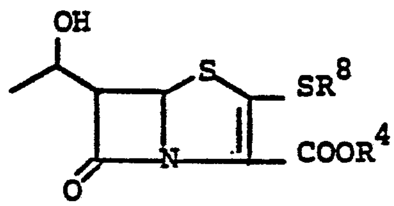

wherein $R^4$ is H, a carboxylate salt cation, removable protecting group, or a pharmaceutically acceptable salt, ester or amide moiety; wherein: $R^8$ is a carbamimidoyl selected from the group consisting of:

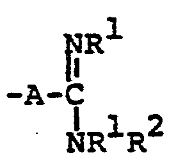
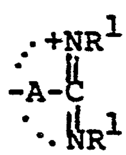
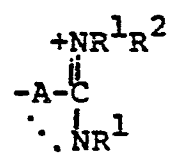
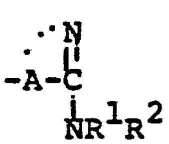
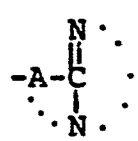
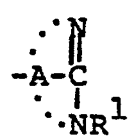
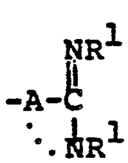
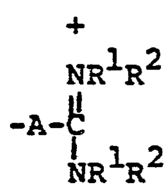
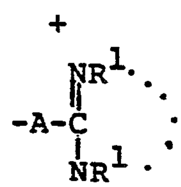
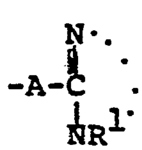
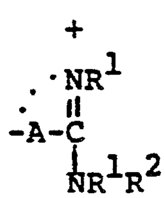
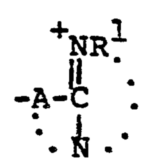

wherein: A is a single, direct bond, or A, the cyclic or acyclic connector, is selected from the group consisting of alkyl, alkenyl, and alkynyl having 1-10 carbon atoms which may be interrupted by a hetero atoms selected from O, S or N, or by a ring such as phenyl, cycloalkyl, cycloalkenyl, heterocyclyl or heteroaryl wherein such cyclic interruptions comprise 3-6 ring atoms selected from C, O, S and N; cycloalkyl, cycloalkenyl having 3-6 carbon atoms; heterocyclyl; heteroaryl; and phenyl; $R^1$ and $R^2$ are independently selected from: hydrogen, $N(R^a)_2$, $OR^a$, ($R^a$ is hydrogen or $C_{1-6}$alkyl), CN, $SO_2NH_2$ and the previously defined, but monovalent, values for the group A; and wherein the dotted lines indicate provision for cyclic structures formed by the joinder of the indicated nitrogen atom and the connector group A and by the joinder of the indicated nitrogen atoms.

3. A compound according to Claim 1 wherein the connecting group A is selected from: a single, direct bond; substituted and unsubstituted: alkyl having from 1 to 6 carbon atoms; cycloalkyl having from 3 to 10 atoms; cycloalkylalkyl wherein the cycloalkyl moiety comprises 3 to 6 carbon atoms and the alkyl moiety comprises 1 to 10 carbon atoms; alkylcycloalkyl wherein the alkyl moiety comprises 1 to 6 carbon atoms and the cycloalkyl moiety comprises 3 to 6 carbon atoms; alkenyl having from 2 to 10 carbon atoms, cycloalkenyl having from 3 to 10 carbon atoms, cycloalkenylalkyl wherein the cycloalkenyl moiety comprises 3 to 10 carbon atoms and the alkyl moiety comprises 1 to 6 carbon atoms; alkynyl having from 2 to 10 carbon atoms; phenyl; naphthyl; arylalkyl and alkylaryl wherein aryl is phenyl and the alkyl has 1 to 6 carbon atoms; heteroalkyl, alkylheteroalkyl, arylheteroalkyl and alkylheteroaryl wherein the heteroatom or atoms are selected from the group of sulfur, oxygen and nitrogen, the alkyl moiety has 1 to 6 carbon atoms, and the aryl moiety is phenyl; heterocyclyl (saturated and unsaturated) comprising mono- and bicyclic structures having 3 to 10 ring atoms wherein one or more of the heteroatoms is selected from oxygen, nitrogen, or sulphur; heterocyclylalkyl wherein heterocyclyl moiety comprises fom 3 to 10 atoms and the alkyl moiety comprises from 1 to 6 atoms; the substituent or substituents relative to the above-named radicals are selected from the group consisting of amino, hydroxyl, cyano, carboxyl, nitro, chloro, bromo,

4. A compound according to Claim 1 wherein the connecting group A is selected from: a single, direct bond, substituted and unsubstituted: straight and branched loweralkyl having from 1 to 6 carbon atoms, cycloalkyl having from 3 to 6 carbon atoms; phenyl; heterocyclyl selected from: thiophene, imidazole, pyridine, tetrazole and furan; alkylheteroalkyl wherein the alkyl moiety comprises 1 to 3 carbon atoms and the heteroatom or atoms are sulfur, oxygen and nitrogen; the substituents relative to the above-named radicals are: amino, hydroxyl, chloro, bromo, fluoro, cyano, carboxyl, alkoxy having from 1 to 3 carbon atoms, mercapto, trifluoromethyl, and alkylthio having from 1 to 3 carbon atoms.

5. A compound according to Claim 1 wherein $-SR^8$ is selected from the group consisting of:

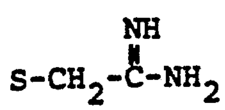

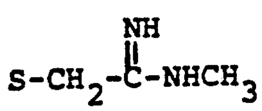

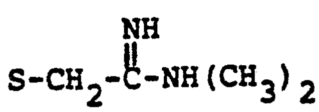

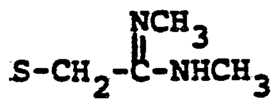

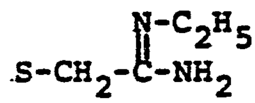

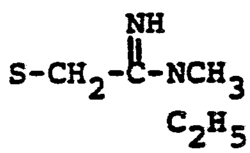

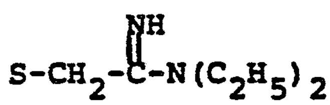

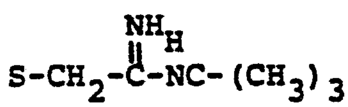

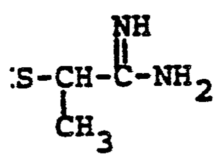

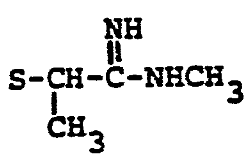

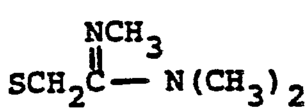

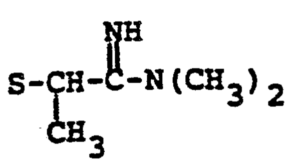

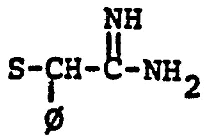

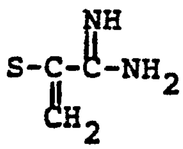

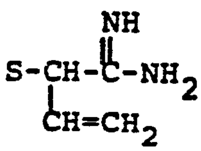

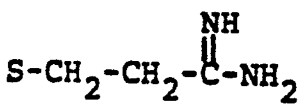

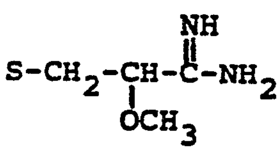

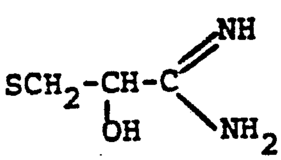

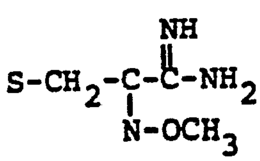

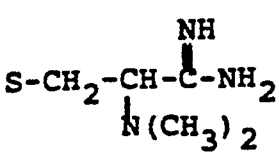

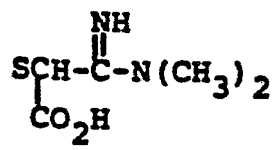

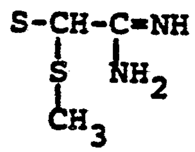

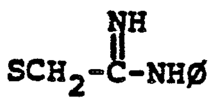

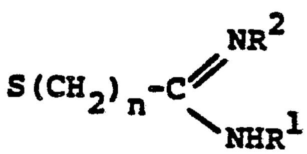

n = 2-5, $R^2$ = H, $CH_3$
$R^1$ = H, $CH_3$

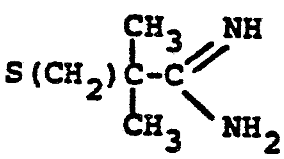

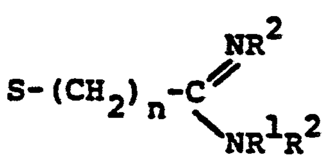

n = 2-5, $R^1$, $R^2$ = H, $CH_3$

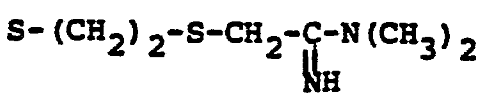

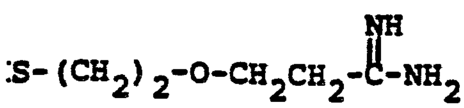

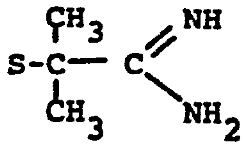

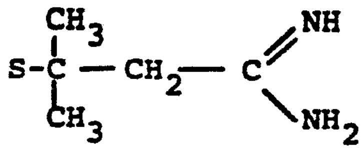

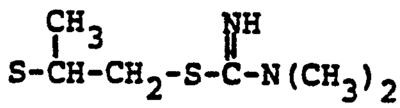

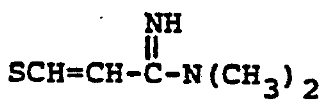

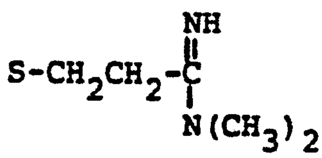

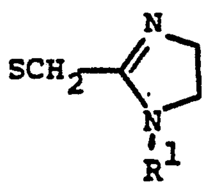

$R^1$=H, $CH_3$

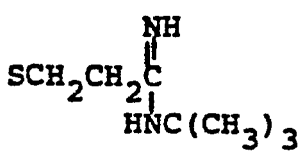

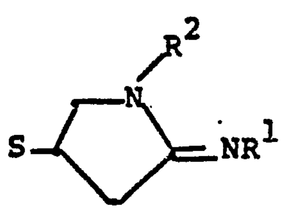

$R^1$ = H, $CH_3$
$R^2$ = H, $CH_3$

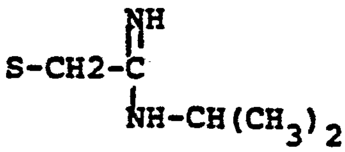

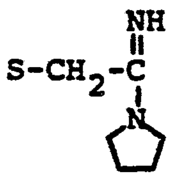

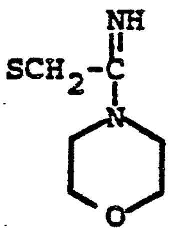

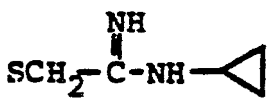

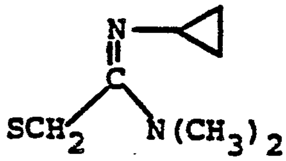

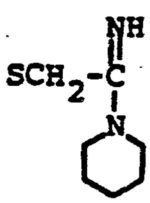

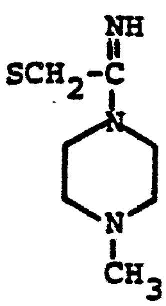

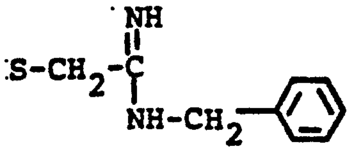

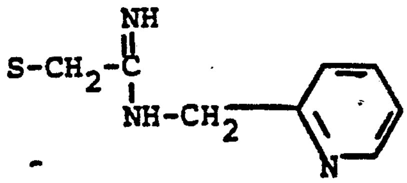

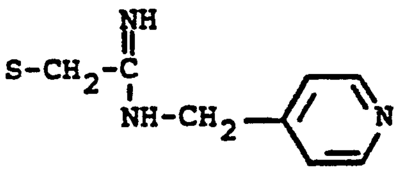

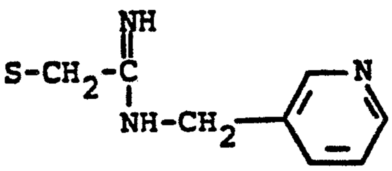

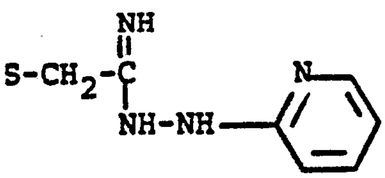

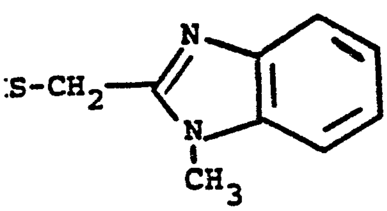

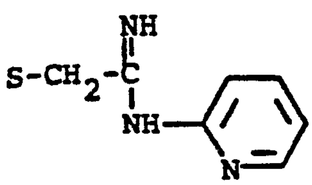

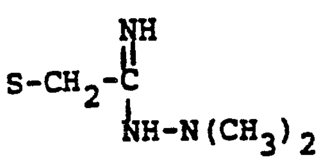

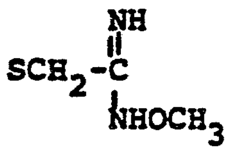

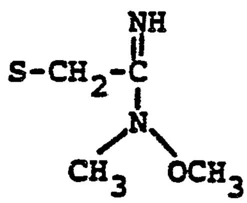

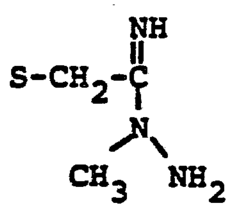

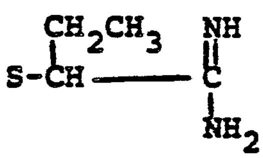

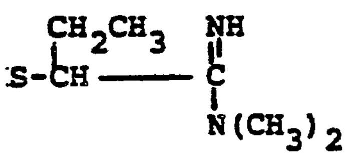

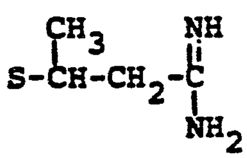

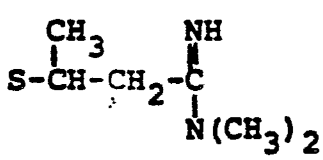

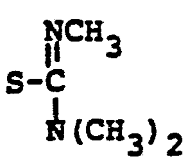

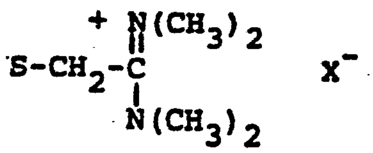

X = any compatible anion

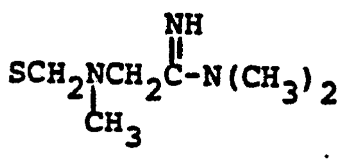

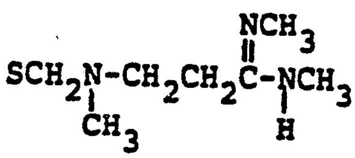

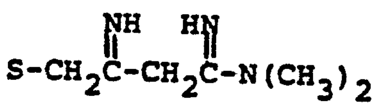

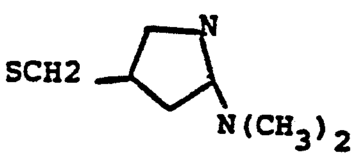

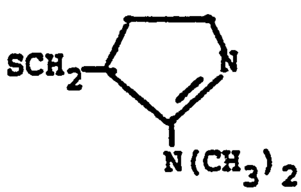

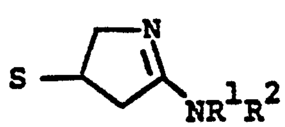

$R^1$ = H, $CH_3$
$R^2$ = H, $CH_3$

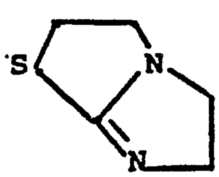

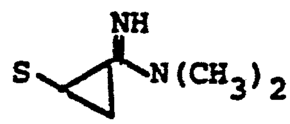

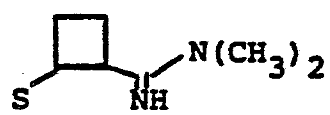

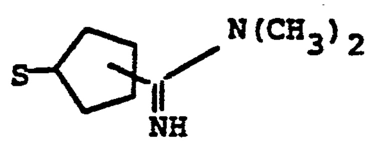

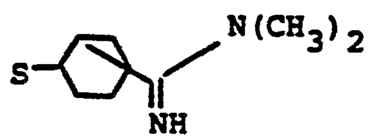

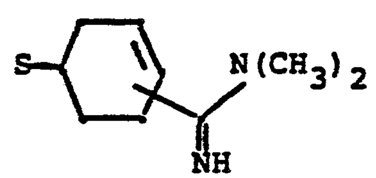

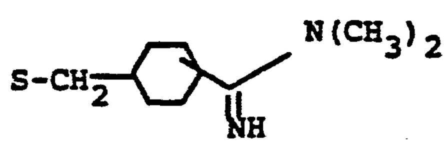

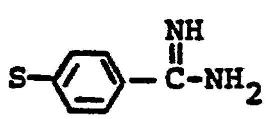

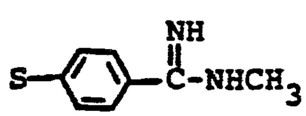

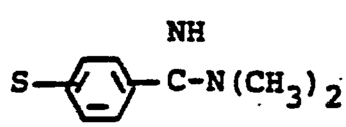

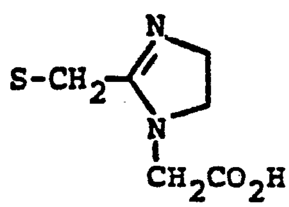

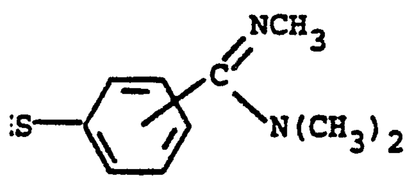

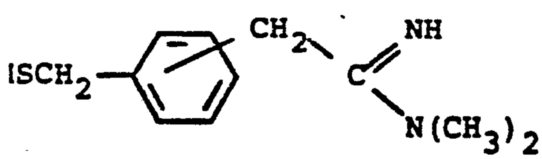

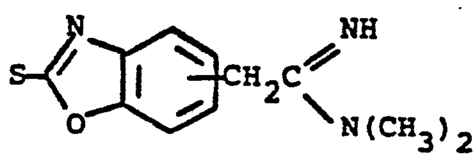

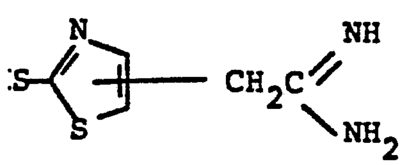

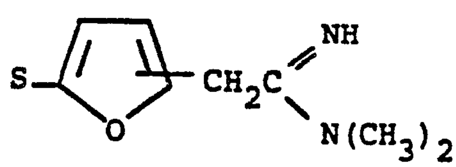

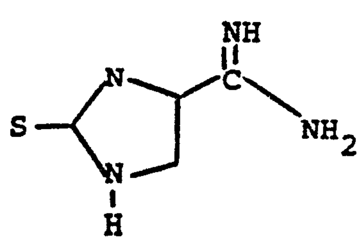

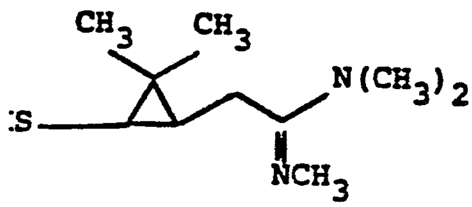

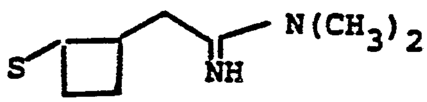

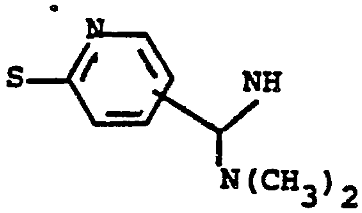

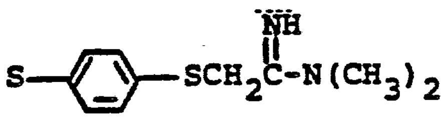

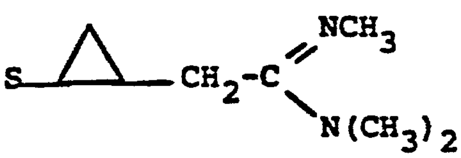

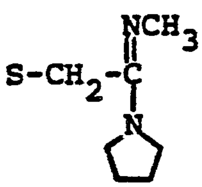

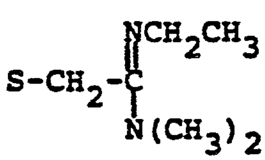

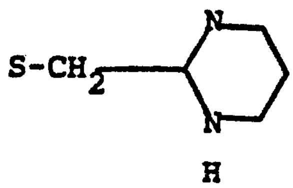

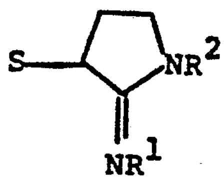

$R^1$ = H, $CH_3$
$R^2$ = H, $CH_3$

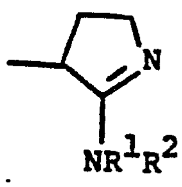

$R^1$ = H, $CH_3$
$R^2$ = H, $CH_3$

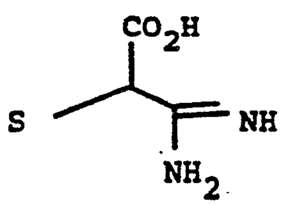

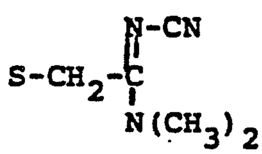

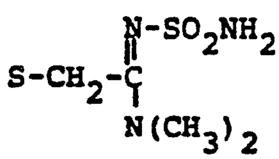

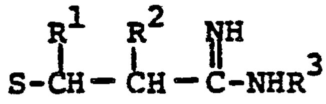

$R^1 = CH_3$
$R^2 = CH_3, N(CH_3)_2, OCH_3$
$R^3 = H, CH_3$

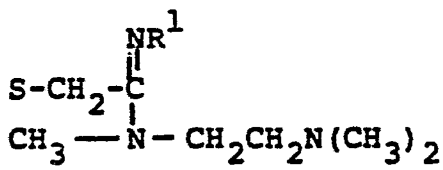

$R^1 = H, CH_3$

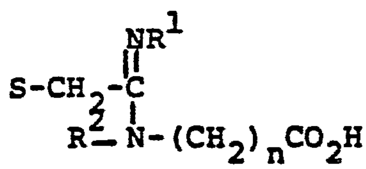

$R^1 = H, CH_3$
$R^2 = H, CH_3$
$n = 1$ or $2$

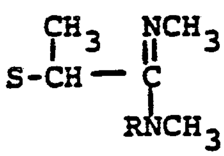

$R = H, CH_3$

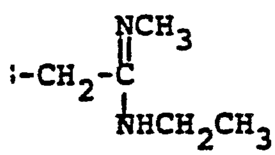

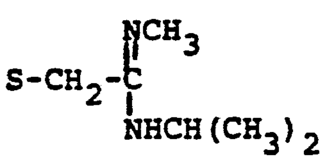

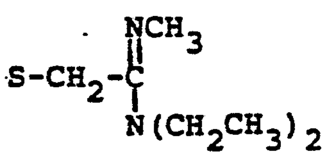

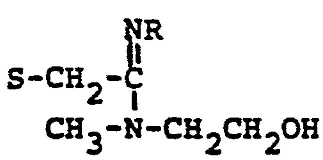

$R = CH_2CH_3, CH_3, H$

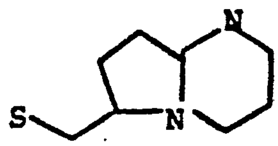

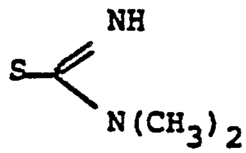

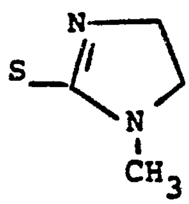

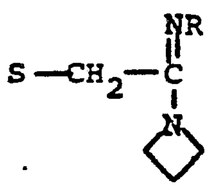

R = H, $CH_3$

6. A process for preparing a compound of the structural formula:

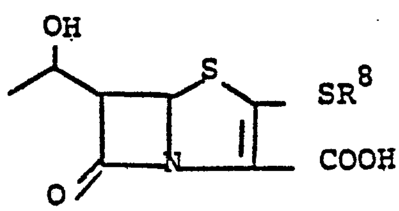

and the pharmaceutically acceptable salt, ester and amide derivatives thereof; wherein: $R^8$ is a carbamimidoyl selected from the group consisting of:

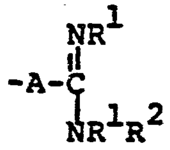 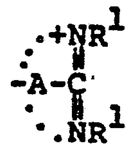 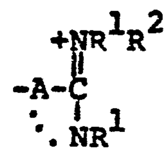

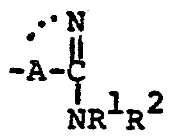 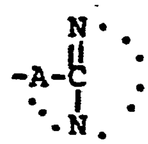 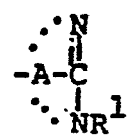

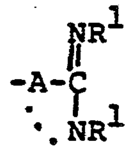 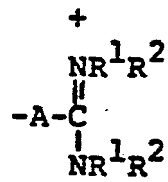 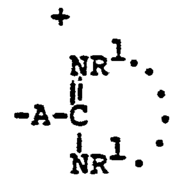

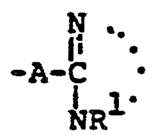 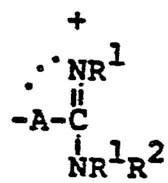 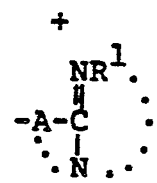

wherein: A is a single, direct bond, or A, the cyclic or acyclic connector, is selected from the group consisting of alkyl, alkenyl, and alkynyl having 1-10 carbon atoms which may be interrupted by a hetero atoms selected from O, S or N, or by a ring such as phenyl, cycloalkyl, cycloalkenyl, heterocyclyl or heteroaryl wherein such cyclic interruptions comprise 3-6 ring atoms selected from C, O, S and N; cycloalkyl, cycloalkenyl having 3-6 carbon atoms; heterocyclyl; heteroaryl; and phenyl; $R^1$ and $R^2$ are independently selected from: hydrogen, $N(R^a)_2$, $OR^a$, ($R^a$ is hydrogen or $C_{1-6}$alkyl), CN, $SO_2NH_2$ and the previously defined, but monovalent, values for the group A; and wherein the dotted lines indicate provision for cyclic structures formed by the joinder of the indicated nitrogen atom and the connector group A and by the joinder of the indicated nitrogen atoms

comprising the step of treating:

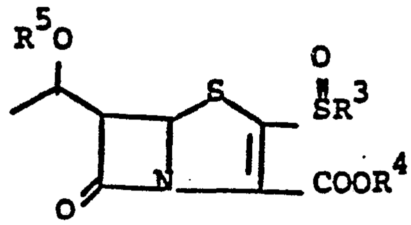

with $HSR^8$; wherein $R^3$ is alkyl, cycloalkyl, or aralkyl; $R^4$ is a protecting group, hydrogen, or a carboxylate salt cation; and $R^5$ is a protecting group or hydrogen followed by the step of deblocking the appropriate protecting groups.

7. An antibiotic composition comprising a therapeutically effective amount of a compound according to Claim 1 and a pharmaceutically effective carrier therefor.

8. A process for preparing a compound of the structural formula:

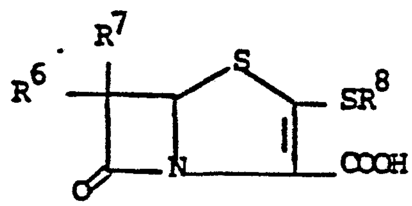

and the pharmaceutically acceptable salt, ester and amide derivatives thereof; wherein: $R^6$ and $R^7$, are independently selected from the group consisting of substituted and unsubstituted: hydrogen alkyl, alkenyl, and alkynyl having 1-10 carbon atoms; cycloalkyl, cycloalkylalkyl, and alkylcyloalkyl having 3-6 carbon atoms in the cycloalkyl ring and

1-6 carbon atoms in the alkyl moiety; aryl, aralkyl, aralkenyl, and aralkynyl wherein the aryl moiety is phenyl and aliphatic portion has 1-6 carbon atoms; heteroaryl, heteroaralkyl, heterocyclyl, and heterocyclylalkyl, wherein the alkyl moiety has 1-6 carbon atoms and the heteroatoms are selected from O, N and S; $R^6$ and $R^7$ may be joined to form a cyclicalkyl having, together with the carbon atom to which they are attached, 3-6 carbon atoms; wherein the substituent or substituents on $R^6$, and $R^7$ are independently selected from chloro, fluoro, hydroxy, bromo, carboxyl, cyano, azido, amino, mono- and dialkylamino (each alkyl having 1-6 carbon atoms, and alkoxyl having 1-6 carbon atoms; and $R^8$ is a carbamimidoyl selected from the group consisting of:

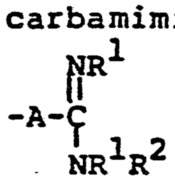 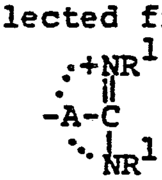 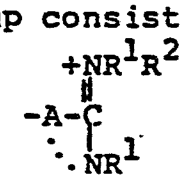

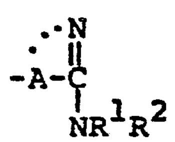 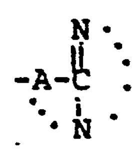 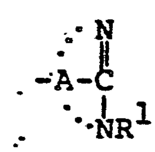

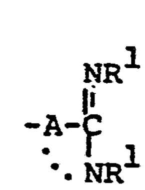 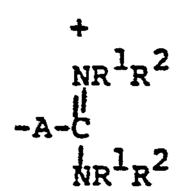 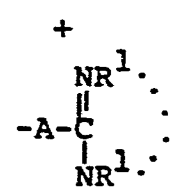

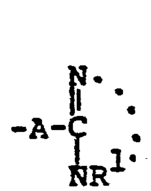 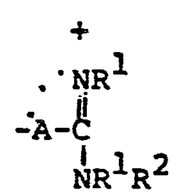 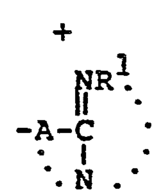

wherein: A is a single, direct bond, or A, the cyclic or acyclic connector, is selected from the group consisting of alkyl, alkenyl, and alkynyl having 1-10 carbon atoms which may be interrupted by a hetero atom selected from O, S or N, or by a ring such as phenyl, cycloalkyl, cycloalkenyl, heterocyclyl or heteroaryl wherein such cyclic interruptions comprise 3-6 ring atoms selected from C, O, S and N; cycloalkyl, cycloalkenyl having 3-6 carbon atoms; heterocyclyl; heteroaryl; and phenyl; $R^1$ and $R^2$ are independently selected from hydrogen and the previously defined, but monovalent, values for the group A; and wherein the dotted lines indicate provision for cyclic structures formed by the joinder of the indicated nitrogen atom and the connector group A and by the joinder of the indicated nitrogen atoms; comprising the step of treating:

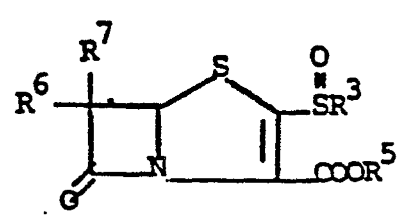

with $HSR^8$; wherein $R^3$ is alkyl, cycloalkyl, or aralkyl; and $R^5$ is a protecting group, hydrogen, or a carboxylate salt cation.

9. An antibiotic composition comprising a therapeutically effective amount of a compound according to Claim 2 and a pharmaceutical effective carrier therefor.

European Patent Office

# EUROPEAN SEARCH REPORT

0087792
Application number

EP 83 10 1923

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | GB-A-2 074 563 (SANKYO)<br>* Claims *<br>--- | 1,7 | C 07 D 499/00<br>A 61 K 31/43 |
| A | EP-A-0 002 210 (MERCK)<br>* Claims 1-3,6 *<br>----- | 1,7 | |
| | | | TECHNICAL FIELDS SEARCHED (Int. Cl. 3) |
| | | | C 07 D 499/00<br>A 61 K 31/00 |

The present search report has been drawn up for all claims

| Place of search | Date of completion of the search | Examiner |
|---|---|---|
| THE HAGUE | 01-06-1983 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82